# EUROPEAN PATENT APPLICATION

(11) **EP 4 310 170 A2**
(43) Date of publication of application: **24.01.2024**
(21) Application number: 23187119.5
(22) Date of filing: 21.07.2023
(51) Int. Cl.: C12M 1/107, C02F 1/24, C02F 3/28, C02F 9/00, C02F 11/04, C12M 1/33, C12M 1/00

(54) **ANAEROBIC WASTE DIGESTION SYSTEM**

(30) Priority: 22.07.2022 US 202217814404
(71) Applicant: Trane International Inc., Davidson, NC 28036 (US)
(72) Inventor: SELMAN, Stanley Scott, Auburn, Alaska, 36830 (US); BROWN, Thomas Garrott, Brookhaven, Georgia, 30319 (US)
(74) Representative: Haseltine Lake Kempner LLP

(57) **Abstract**

A system and method are each directed to generating a biogas including methane by bacterial digestion of waste materials under anaerobic conditions. The waste digestion system includes three processing stages and a biogas production unit. The waste material is provided in the form of a water based slurry including solid particles having a distribution of particle sizes. The three processing stages are configured to remove and/or process the solid particles in the slurry, such that the biogas production unit receives a feedstock enriched in solid particles having a particle size suited for efficient digestion. The method generally includes processing the waste material in the three processing stages, digesting the waste material under anaerobic conditions, thereby generating the biogas.

## Description

### TECHNICAL FIELD

The present disclosure generally relates to systems and methods for processing various waste materials, and in particular, to systems and methods for generating methane from such waste materials by anaerobic digestion.

### BACKGROUND

Anaerobic digestion is a naturally occurring biological process which finds utility in the processing of various waste streams, including municipal waste water. Particularly, anaerobic digestion is useful in removing undesirable organisms and reducing turbidity in municipal waste water. Anaerobic digestion of waste sludge is beneficial in reducing or removing volatile organic components, undesirable odors, and pathogens in waste sludge, providing useful by-products and a reduced sludge handling and disposal costs. The anaerobic digestion process is also capable of breaking down hard-to-digest natural organic compounds, including toxic solvents, degreasers, cleaners, paints, and coating materials. Thus, commercial anaerobic digesters have recognized benefits, chiefly in providing cost savings in reducing disposal costs, which are proportionate to the amount of waste processed.

The process of anaerobic digestion also naturally produces biogas rich in methane. Methane gas, the main component of natural gas, accordingly has potential commercial value as a fuel. However, methane production has not been a compelling reason to utilize anaerobic digesters to process waste materials, as it has historically been generally cheaper to obtain natural gas from oil and gas wells. Accordingly, development of commercial anaerobic digesters and digestion processes have not focused on optimization of this aspect.

In view of the potential of anaerobic digestion to generate valuable methane gas while simultaneously reducing waste disposal costs, systems and processes for anaerobically digesting waste and producing methane would find utility across numerous industries. Generally, any enterprise producing large volumes of waste digestible by bacteria under anaerobic conditions would benefit from the opportunity to generate revenue from the sale of efficiently generated methane while enjoying the savings afforded by the concomitant reduction in waste disposal costs. Accordingly, it would be desirable in the art to provide systems and processes for efficient anaerobic digestion of a wide variety of waste materials while simultaneously providing methane in an economically viable manner.

### SUMMARY

The present disclosure generally provides a waste digestion system configured to produce a biogas comprising methane from the digestion of certain waste materials under anaerobic conditions. Further provided are methods of generating a biogas comprising methane from the digestion of certain waste materials under anaerobic conditions. The design of prior anaerobic digesters or systems exhibit various liabilities including the need to replenish bacteria, lengthy hydraulic residence times (e.g., up to three weeks), sensitivity to clogging, and low efficiencies. In all prior art designs, the digester requires periodic cleaning to remove settled solids otherwise, efficiency will drop and the system will ultimately fail. The system of the present disclosure overcomes these issues by virtue of the three processing stages and unique fixed film digester design. The disclosed system provides surprising advantages, such as short hydraulic residence time (e.g., typically around 3 days), a higher rate of conversion of solids to methane (e.g., approximately seven times faster than prior systems), and/or high efficiency.

In an embodiment, a waste digestion system is configured to generate a biogas from a waste stream including a waste material by bacterial digestion of the waste material under anaerobic conditions. The biogas includes methane. The waste stream includes a first water-based slurry including solid particles. The solid particles include non-digestible solid particles, small size solid particles that are solid particles having a particle size suitable for efficient anaerobic bacterial digestion, and large size solid particles that are solid particles having a particle size larger than the particle size suitable for efficient anaerobic bacterial digestion. The waste digestion system includes a first processing stage, a second processing stage, a third processing stage, and a biogas production unit. The first processing stage is configured to remove the non-digestible solid particles from the first water-based slurry and to form a second water-based slurry including the small size solid particles and large size solid particles. The second processing stage is in fluid communication with the first processing stage to receive the second water-based slurry. The second processing stage is configured to reduce the particle size of at least a portion of the large size solid particles and to form a third water-based slurry. The third processing stage is in fluid communication with the second processing stage to receive the third water-based slurry. The third processing stage includes a dissolved gas flotation (DGF) separator having at least one separation zone including a bubbler. The DGF separator is configured to utilize non-oxygenated gas to remove a remaining portion of the large size solid particles from the third water-based slurry, such that the third processing stage discharges a fourth water-based slurry enriched in the small size solid particles. The biogas production unit is connected in fluid communication with the DGF separator to receive the fourth water-based slurry as a feedstock. The biogas production unit is configured to anaerobically digest the small sized solid particles in the feedstock forming the biogas, wastewater, and settled solids. The biogas production unit is configured to discharge the biogas as a product, to discharge the wastewater as an effluent, and to discharge the settled solids as a fifth water-based slurry. The biogas production unit includes at least one anaerobic digester with a first bio-substrate for growing bacteria under anaerobic conditions to produce the biogas and a first heat exchanger configured to heat the feedstock using heat in the effluent.

In an embodiment, the waste digestion system also includes one or more mechanical particle size reducers configured to mill or grind at least a portion of the larger solid particles.

In an embodiment, the first processing stage includes at least one of a macerator and a grinder configured to reduce the particle size of at least a portion of the solid particles.

In an embodiment, the second processing stage includes a thermal steam explosion (TSE) unit configured to hydrolyze at least a portion of the large size solid particles.

In an embodiment, the second processing stage includes an electrocoagulation (EC) unit. The EC unit is configured to perform one or more of the following: electrochemically hydrolyze at least a portion of the large size solid particles, electrochemically destabilize at least a portion of the large size solid particles, and cause at least a portion of the large size solid particles to settle out of the second water-based slurry.

In an embodiment, the at least one anaerobic digester includes a second bio-substrate. The first bio-substrate and the second bio-substrate are configured to independently receive, respectively, a first feedstock stream and a second feedstock flow stream of the feedstock, and wherein the first feedstock flow stream and the second feedstock flow stream have at least one of a different flow rate and a different distribution of particle sizes.

In an embodiment, the waste digestion system also includes at least one compressor, a compressor exchange fluid for adsorbing waste heat from the compressor, and a second heat exchanger. The second heat exchanger is configured to heat the feedstock using the compressor heat exchange fluid heated by the waste heat of the compressor.

In an embodiment, the waste digestion system also includes a boiler, a boiler heat exchange fluid for absorbing heat from the boiler, and a third heat exchanger. The third heat exchanger is configured to heat the feedstock using the boiler heat exchange fluid heated by the boiler.

In an embodiment, the at least one heat exchanger is configured to provide an anaerobic digester operating temperature in a range from at or about 68 to at or about 140°F.

In an embodiment, the waste material includes one or more of waste food, municipal sewage waste, animal waste from farming operations, industrial organic waste, and fat, oil, and grease (FOG) waste.

In an embodiment, the waste digestion system also includes an equalization tank, at least one slurry pump, and a solids tank. The at least one slurry pump is for reducing particle size of the solid particles by hydraulic shear. The at least one slurry pump is in fluid communication with the equalization tank. The first processing stage is in fluid communication with the equalization tank and the at least one slurry pump. The solids tank is in fluid communication with the first processing stage. The solids tank is configured to receive a portion of the large solid particles.

In an embodiment, the particle size suitable for efficient anaerobic bacterial digestion is less than about 200 µm.

In an embodiment, the first processing stage is further configured to remove from the first water-based slurry the solid particles having a particle size greater than about 750 µm, and is further configured to mechanically reduce particle size of said solid particles.

In an embodiment, the waste digestion system also includes a process control configured to control the waste digestion system.

In an embodiment, a method is directed to generating a biogas from a waste stream including a waste material by bacterial digestion of the waste material under anaerobic conditions. the biogas includes methane. The waste stream includes a first water-based slurry including solid particles. The solid particles include non-digestible solid particles, small sized solid particles that are solid particles having a particle size suitable for efficient anaerobic bacterial digestion, and large size solid particles that are solid particles having a particle size larger than the particle size suitable for efficient anaerobic bacterial digestion. The method includes providing a first processing stage downstream of a source of waste, receiving the first water-based slurry from the source of waste, removing by the first processing stage the non-digestible solid particles from the first water-based slurry, thereby forming a second water-based slurry including the small size solid particles and the large size solid particles, and allowing passage of the second water-based slurry through the first processing stage. The method also includes providing a second processing stage downstream of and in fluid communication with the first processing stage, receiving the second water-based slurry from the first processing stage, reducing by the second processing stage the particle size of at least a portion of the large size solid particles, thereby forming a third water-based slurry, and allowing passage of the third water-based slurry through the second processing stage. The method also includes providing a third processing stage downstream of and in fluid communication with the second processing stage and receiving the third water-based slurry from the second processing stage. The third processing stage includes a dissolved gas flotation (DGF) separator comprising a bubbler. The DGF separator including at least one separation zone. The method also includes removing and retaining a remaining portion of the large size solid particles in the at least one separation zone using non-oxygenated gas, thereby forming a fourth water-based slurry enriched in the small size solid particles, and allowing passage of the fourth water-based slurry through the third processing stage. The method also includes providing a biogas production unit downstream of and in fluid communication with the DGF separator and receiving the fourth water-based slurry from the third processing stage as a feedstock to the biogas production unit. The biogas production unit includes at least one anaerobic digester including at least a first bio-substrate for growing bacteria under anaerobic conditions and a first heat exchanger. The method also includes forming the biogas, wastewater, and settled solids by allowing the bacteria growing on the bio-substrate to anaerobically digest the small size solid particles in the fourth water-based slurry, allowing the biogas to exit the biogas production unit as a product, allowing the wastewater to exit the biogas production unit as an effluent, and allowing the settled solids to exit the biogas production unit as a fifth water-based slurry. The first heat exchanger is in fluid communication with the feedstock and is in separate fluid communication with the effluent. The first heat exchanger is configured to provide heat to the feedstock using heat in the effluent.

In an embodiment, removing the non-digestible solid particles from the first water-based slurry includes passing the first water-based slurry through one or more of the following: a passive screen, a vibratory screen, a settling chamber, a centrifugal separator, a clarifier, and a screw press.

In an embodiment, the method includes removing from the first water-based slurry the solid particles having a particle size greater than about 750 µm, and mechanically reducing the particle size of said solid particles.

In an embodiment, the second processing stage includes a thermal steam explosion (TSE) unit configured to hydrolyze at least a portion of the large sized solid particles. The method includes passing the second water-based slurry through the TSE unit.

In an embodiment, the second processing stage includes an electrocoagulation (EC) unit. The method includes passing the second water-based slurry through said EC unit, thereby performing one or more of the following: electrochemically hydrolyzing at least a portion of the large size solid particles, electrochemically destabilizing at least a portion of the large size solid particles, and causing at least a portion of the large size solid particles to stratify and settle out of the second water-based slurry.

In an embodiment, the method includes providing one or more mechanical particle size reducers configured to at least one of mill and grind solid particles to have a smaller size. The method also includes one or more of milling and/or grinding, with the one or more mechanical particle size reducers, at least a portion of the large size solid particles by passing said large size solid particles through the one or more mechanical particle size reducers.

### BRIEF DESCRIPTION OF THE DRAWINGS

Having thus described aspects of the disclosure in the foregoing general terms, reference will now be made to the accompanying drawing, which is not necessarily drawn to scale. The drawings are exemplary only, and should not be construed as limiting the disclosure.
FIG. 1 shows a schematic diagram of a waste digestion system, according to a non-limiting embodiment.
FIG. 2 shows a schematic diagram of a first processing stage, according to a non-limiting embodiment.
FIG. 3 shows a schematic diagram of a second processing stage, according to a non-limiting embodiment.
FIG. 4 shows a schematic diagram of a third processing stage, according to a non-limiting embodiment.
FIG. 5 shows a schematic diagram of a biogas production unit, according to a non-limiting embodiment.
FIG. 6 is a flowchart illustrating various operations in a method for generating a biogas comprising methane from a waste stream, according to a non-limiting embodiment.

### DETAILED DESCRIPTION

U.S. Patent Application Ser. No. 16/704,750, now U.S. Patent No. 10,899,640, is herein incorporated by reference in its entirety and for all purposes.

The present disclosure will now be described more fully hereinafter with reference to example embodiments thereof. These example embodiments are described so that this disclosure will be thorough and complete, and will fully convey the scope of the disclosure to those skilled in the art. Indeed, the disclosure may be embodied in many different forms and should not be construed as limited to the embodiments set forth herein; rather, these embodiments are provided so that this disclosure will satisfy applicable legal requirements.

Although specific terms are employed, the terms are used in a descriptive sense only and not for purposes of limitation. Embodiments of systems and methods have been described in considerable detail with specific reference to the illustrated embodiments. However, it will be apparent that various modifications and changes can be made within the spirit and scope of the embodiments of systems and methods as described in this specification, and such modifications and changes are to be considered equivalents and part of this disclosure.

The following includes definitions of various terms and phrases used throughout this specification.

The use of the words "a" or "an" when used in conjunction with the term "comprising," "including," "containing," or "having" in the claims or the specification may mean "one," but it is also consistent with the meaning of "one or more," "at least one," and "one or more than one."

The words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. The process of the present disclosure can "comprise," "consist essentially of," or "consist of" particular ingredients, components, compositions, etc., disclosed throughout the specification.

The terms "about" or "approximately" are defined as being close to as understood by one of ordinary skill in the art. In an embodiment, these terms are defined as being within 10%. In an embodiment, these terms are defined as being within 5%. In an embodiment, these terms are defined as being within 1%. In an embodiment, these terms are defined as being within 0.5%.

As used herein, the term "comminution" refers to the reduction of solid materials from one average particle size to a smaller average particle size, by crushing, grinding, cutting, vibrating, or other processes, so as to reduce the size and to increase the surface area of solids.

As used herein, the term "RNG" refers to Renewable Natural Gas, which is pipeline quality, 95%-98% pure methane.

As used herein, the term "PFAD" refers to parallel flow Anaerobic Digester, which is a continuous feed, fixed film design digester.

As used herein, the term "DGF" refers to Dissolved Gas Flotation.

As used herein, the term "EC" refers to Electrocoagulation, which is electrochemically induced hydrolysis and particle separation.

As used herein, the term "VS" refers to Volatile Solids, which is the amount of OM (organic matter) in a waste slurry. Volatile Solids analysis determines the total amount of OM in the slurry on a dry mass basis. In some embodiments, the VS content of a slurry is 60 percent or more on a dry mass basis, as non-volatile solids do not contribute to methane production.

As used herein, the term "OD" refers to Oxygen Demand, which is used to estimate the energy content of organic matter. Organic matter with high energy content produces more methane than OM with low energy content.

As used herein, the term "COD" refers to Chemical Oxygen Demand, which is generally used to determine OD (oxygen demand) for anaerobic digestion. A COD test measures OM in the absence of oxygen. COD will vary by dilution with water, but is often greater than 15,000 mg/ml, that is, mg of OM per ml of slurry.

As used herein, the term "OLR" refers to Organic Loading Rate, which is how much VS is fed into or loaded per day into a digester. OLR and methane generation are directly proportional.

As used herein, the term "SRT" refers to Solids Residence Time, and is the length of time the solid particles stay in the digester. SRT is directly related to Organic Loading Rate, due to the high concentration of organics present in the solids entering the digester.

As used herein, the term "HRT" refers to Hydraulic Residence Time, and is the digester's wetted volume divided by the flow rate of the waste slurry passing through the digester. It represents the bulk measure of time between influent flow entering and effluent flow exiting the digester.

As used herein, the term "KOP" refers to Knock Out Pot, a process apparatus that removes excess water from the biogas. Also known as a flash drum, the KOP prevents entrained droplets of water, liquids, and particulates from reaching downstream process equipment.

As used herein, the term "EQ" refers to equalization. EQ Pit refers to a concrete holding pit for eventual discharge to an EQ tank.

As used herein, the term "water-based slurry" refers to a suspension in water of solid waste particles having a particle size distribution (e.g., a combination of large, mid-sized, and small solid waste particles).

As used herein, the term "feedstock" refers to wastewater that has passed through the three processing stages as described herein, and has an average particle size ideal for anaerobic digestion (e.g., effluent (fourth water-based slurry) leaving the third processing stage and entering the biogas production unit is feedstock).

As used herein, the term "PET" refers to polyethylene terephthalate.

As used herein, the term "instruments" are defined as follows: LSH refers to Level Sensor High; LSL refers to Level Sensor Low; pH refers to Acid/Base Sensor; TI refers to Temperature Indicator; PI refers to Pressure Indicator; and DP refers to Differential Pressure Sensor.

A micron (µ) or micrometer (µm) is a unit of linear measurement equal to one thousandth of a millimeter, or one millionth of a meter.

As used herein, the word "exemplary" means "serving as an example, instance, or illustration." Any embodiment described herein as "exemplary" is not necessarily to be construed as preferred or advantageous over other embodiments. The word "example" may be used interchangeably with the term "exemplary."

### Waste Digestion System

In an embodiment, a waste digestion system is configured to generate a biogas comprising methane from a waste stream. The system generally includes a first processing stage, a second processing stage, a third processing stage, a biogas production unit, and a process control. Each of the processing stages are configured to receive a water-based slurry, and to gradually provide a final feedstock enriched in solid particles having a particle size suitable for anaerobic digestion in the biogas production unit. Each of the stages and components of the system, and the system as a whole, are further described herein below.

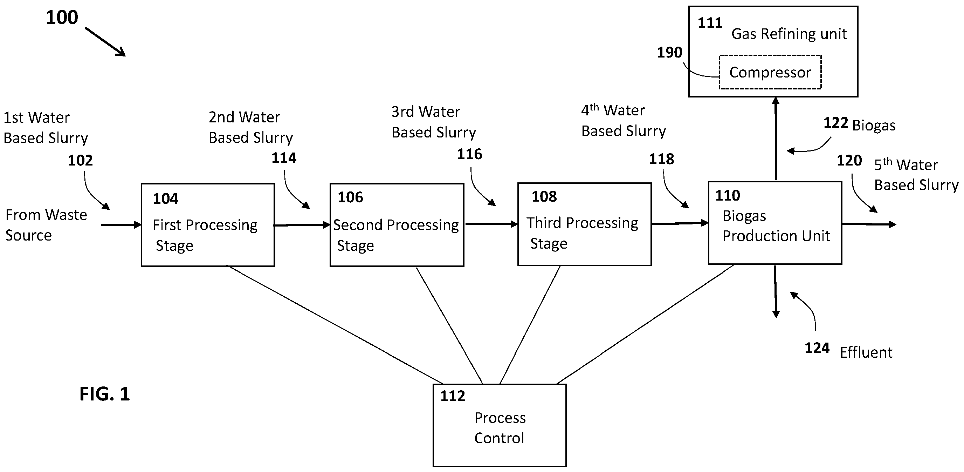

A schematic illustration of a waste digestion system 100 according to an embodiment is provided in FIG. 1. With reference to FIG. 1, the waste digestion system 100 is configured to generate a biogas including methane from a waste stream including a waste material by bacterial digestion of the waste material under anaerobic conditions. The waste digestion system 100 can include a first processing stage 104, a second processing stage 106, a third processing stage 108, and a biogas production unit 110. For example, the first processing stage 104, the second processing stage 106, the third processing stage 108, and the biogas production unit 110 are fluidly connected in series. Each of the stages 104, 106, 108 and the biogas production unit 110 are described in more detail below.

### Waste material and first water-based slurry

The waste material present in the waste stream may vary according to the source of the waste stream, but generally includes organic materials which are digestible under anaerobic conditions. Examples of such waste materials include, but are not limited to, food wastes, organic chemical wastes, urine, manure, fats, oils, greases, cellulosic materials, and the like. Sources of such waste materials include, but are not limited to, industrial and manufacturing processes, municipal sewage, cattle farms, dairy farms, and other farming-related sources, breweries, paper mills, meat processing facilities, industrial food processing and packing plants, and the like.

In an embodiment, the waste material includes one or more of waste food, municipal sewage waste, animal waste from farming operations, industrial organic waste, and fat, oil, and grease ("FOG") waste.

In an embodiment, the source of the waste material is one or more of a brewery and distillery, and the waste material includes one or more of grain(s), hops, yeast, and wort.

In an embodiment, the source of the waste material is a farming operation, and the waste material includes one or more of plant material, urine, and manure. In an embodiment, the farming operation may be an animal farm (e.g., a dairy farm, a cattle farm, a pig farm, a chicken farm, or the like), an agricultural farm, or a combination thereof. In an embodiment, the farming operation can be an agricultural farm.

In an embodiment, the source of the waste material is a paper mill, and the waste material includes one or more cellulosic material(s) and effluent(s) associated with the paper making and refining process.

In an embodiment, the source of the waste material is a food-related industry, and the waste material comprises one or more of meat, fish, poultry, vegetable(s), fat(s), oil(s), and grease(s).

In an embodiment, the source of the waste material is a chemical industry, and the waste material includes one or more organic chemical substances. For example, the one or more organic chemical substances can include, but not limited to, solvent(s), paint(s), coating(s), dye(s), pigment(s), pharmaceutical(s), and the like.

Generally, the waste material is provided in the form of a first water-based slurry comprising solid particles have a distribution of particle sizes. The term "distribution of particle sizes" refers to solid particles having a variety of particle sizes being present in the slurry, with the individual particle sizes and the distribution thereof based on the source and nature of the waste material, and accordingly, the nature of the waste material as described herein above. Generally, a portion of the solid particles (e.g., a first portion of the solid particles) have a particle size larger than a particle size suitable for efficient anaerobic bacterial digestion, some portion of the solid particles (e.g., a second portion of the of solid particles) have a particle size suitable for efficient anaerobic bacterial digestion, and some portion of the solid particles (e.g., a third portion of the solid particle) are non-digestible. The rate of anaerobic bacterial digestion is determined at least in part by the particle size of digestible particles, with smaller particles generally resulting in more rapid digestion and methane production. The size of individual particles in the first water-based slurry may vary from less than 1 micron up to approximately 1500 microns or more. While large particles may still be digested, it is generally preferable to provide to the biogas production unit a feedstock enriched in solid particles having a particle size suitable for efficient digestion to optimize the anaerobic digestion process and produce the maximum amount of methane yield per pound of feedstock. Particularly, particle sizes of at or about 200 microns or less meet this criterion. In an embodiment, the particle size suitable for efficient anaerobic bacterial digestion is less than at or about 150 microns. In an embodiment, the particle size suitable for efficient anaerobic bacterial digestion is from at or about 10 to at or about 150 microns. In an embodiment, the particle size suitable for efficient anaerobic bacterial digestion is from at or about 10 to at or about 75 microns.

With continued reference to FIG. 1, the waste stream entering the system 100 includes a first water-based slurry 102 that includes solid particles having a distribution of particle sizes. A portion of the solid particles (e.g., a first portion of the solid particles) have a particle size larger than a particle size suitable for efficient anaerobic bacterial digestion, some portion of the solid particles (e.g., a second portion of the solid particles) have a particle size suitable for efficient anaerobic bacterial digestion, and some portion of the solid particles (e.g., a third portion of the solid particles) are non-digestible. The system 100 includes three processing stages to remove non-digestible solid particles and process the solid particles having a particle size larger than a particle size suitable for efficient anaerobic bacterial digestion. The system 100 is able to provide feedstock enriched in solid particles having a particle size suitable for efficient anaerobic bacterial digestion.

### First processing stage

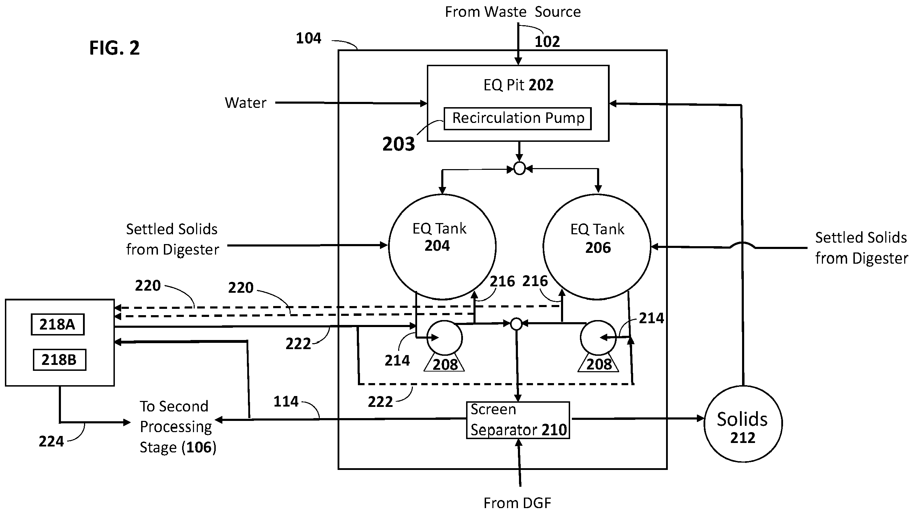

With continued reference to FIG. 1, the system 100 includes a first processing stage 104. The first processing stage 104 is configured to remove the non-digestible solid particles from the first water-based slurry 102. A schematic illustration of a first processing stage 104 according to an embodiment is provided in FIG. 2. With reference to FIG. 2, in an embodiment, the first processing stage 104 includes one or more of the following: a passive screen, a vibratory screen, a settling chamber, a centrifugal separator, a clarifier, and a screw press. For the avoidance of doubt, reference to one or more is intended to encompass both combinations of any two or more of the listed devices, as well as multiples of each listed device (e.g., two or more passive screens, two or more vibratory screens, etc.). As shown in FIG. 2, the first processing stage 104 includes a screen separator 210. In an embodiment, the screen separator 210 includes one or more of a passive screen, a vibratory screen, and a turbo-screen clarifier.

The nature of the non-digestible particles may vary depending on the source and nature of the waste material. One non-limiting example of a non-digestible solid particle is sand, which is commonly used in dairy farms for bedding cows. In an embodiment, the waste material is dairy cattle waste, and the waste digestion system includes a sand lane for receiving the dairy cattle waste. The sand lane includes bedding sand and is configured to receive water from a water source, forming a sand-manure slurry. In an embodiment, the system 100 is configured to separate the bedding sand from the sand-manure slurry, forming the first water-based slurry 102. Such separation may occur in a separate pre-processing stage, or may be performed within the first processing stage 104. Accordingly, in an embodiment as shown in FIG. 2, the system 100 includes an equalization (EQ) pit 202 connected in fluid communication with the sand lane (not shown), and one or more equalization tanks 204, 206. The equalization pit 202 is configured to receive the sand-manure slurry and to allow passage of the first water-based slurry therethrough to the equalization tank(s) 204, 206. In an embodiment, the EQ pit 202 may include a recirculation pump 203 within the EQ pit 202. The recirculation pump 203 can be configured to chop or macerate larger solid particles within the first water-based slurry 102 within the EQ pit 202.

In an embodiment, a high volume of recycled water from the water source (e.g., a lagoon and/or precipitation falling on livestock yards and outdoor confinement areas) and urine mixed with sand and the solid manure from the cattle stall floor, and the mixture of sand, urine, and solid manure is directed into the sand lane (e.g., a long, narrow trough). The water dilutes the manure and allows the sand to settle out on the bottom of the sand lane, forming the first water-based slurry 102. The sand is periodically removed, washed, dried, and reused as bedding. The sand lane is one non-limiting example of a method of sand separation. Other methods of sand separation are known in the art, and it should be appreciated a different method of sand separation may be employed in other embodiments.

In some embodiments, the first water-based slurry 102 flows by gravity into the EQ pit 202. From the EQ pit 202, the slurry 102 enters the EQ tank(s) 204, 206 by gravity flow. The disclosed system 100 generally relies primarily upon gravity flow to convey slurry and other fluids from any stage or tank at a given elevation, to a downstream stage or tank situated at a lower elevation. It should be appreciated that pump(s) may be utilized at any point in the system where gravity is insufficient to maintain the process flow.

In an embodiment, the waste digestion system 100 (e.g., the first process stage 104) includes at least one slurry pump 208 in fluid communication with an EQ tank(s) 204, 206. With continued reference to FIG. 2, in some embodiments, the first processing stage 104 includes two EQ tanks 204, 206. Generally, when employing at least two EQ tanks, a first EQ tank 204 is filling up while a second EQ tank 206 is discharging (e.g., through a slurry pump inlet 214 to a slurry pump 208). When the flow is changed, the second EQ tank 206 is filling up while the first EQ tank 204 is discharging (e.g., through a slurry pump inlet 214 to the slurry pump 208). Alternating the flow path in this manner provides a nearly continuous flow for downstream processing. A slurry pump 208 may be provided for each EQ tank 204, 206 as shown in FIG. In an embodiment, the single slurry pump 208 may be used for multiple EQ tanks 204, 206. As shown in FIG. 2, output from the slurry pump(s) 208 is sent to the screen separator 210. In an embodiment, a portion of the slurry pump output may be diverted through slurry recirculation 216 back to the EQ tank(s) 204/206. This serves to agitate the slurry, so as to prevent settling out of solids in the EQ tank(s).

In an embodiment, the slurry pump 208 is configured for reducing particle size of the solid particles by hydraulic shear. The solids are subjected to high shear forces within the slurry pump 208, breaking down large particles by hydraulic shear and releasing organic particles, thereby increasing available COD.

In an embodiment, the waste digestion system 100 may further include a solids tank 212 in fluid communication with the first processing stage 104. The solids tank 212 is configured to receive solid particles larger than those suitable for efficient anaerobic bacterial digestion. As shown in FIG. 2, the solids tank 212 can be configured to receive said particles from the screen separator 210.

In an embodiment, the first processing stage 104 can further include a clarifier (not shown). A clarifier may be included, for example, when the waste material is municipal sewage waste.

In an embodiment, the first processing stage 104 includes a macerator and/or a grinder configured to reduce the particle size of at least some portion of the solid particles present in the second water-based slurry. In an embodiment, the recirculation pump 203 may also be configured as a macerator that is configured to macerate larger solid particles in the slurry within the EQ pit 202.

In an embodiment, the first processing stage 104 can be configured to remove slurry solids that have a high phosphorus concentration, and optionally direct such high phosphorus solids to a high phosphorus solids tank (not shown).

Following the processing occurring in the first processing stage 104 (e.g., separation, maceration, hydraulic shear), the product is a second water-based slurry 114. As shown in Figures 1 and 2, the second water-based slurry 114 is discharged from the first processing stage 104 (e.g., from the screen separator 210 of the first processing stage 104). The second water-based slurry 114 includes solid particles having a particle size suitable for efficient anaerobic bacterial digestion and solid particles having a particle size larger than the particle size suitable for efficient anaerobic bacterial digestion. In an embodiment, up to approximately 70% of the larger suspended solids (e.g., solid particles of >750 microns) are separated from the first water-based slurry 102, providing an enrichment with respect to particles suitable for efficient anaerobic digestion. The large solids removed may be removed to a solids tank 212 as described above, or may be subject to further processing to reduce particle size (e.g., recycled through a slurry pump, grinder mill, or macerator). For example, in an embodiment, the first processing stage 104 can be configured to remove solid particles having a particle size greater than about 750 µm from the first water-based slurry 102, and configured to mechanically reduce particle size of said solid particles.

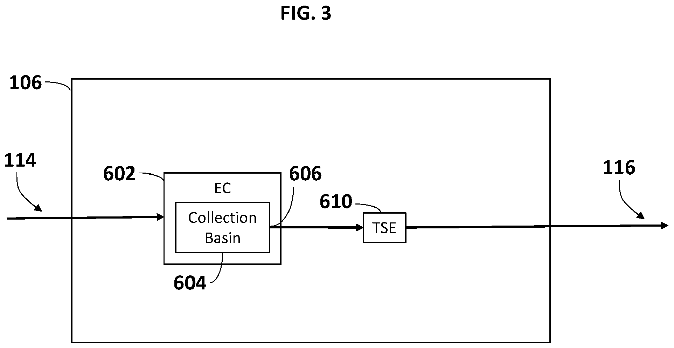

The system 100 (e.g., the first processing stage 104) includes a grinder mill 218A and/or a Thermal Steam Explosion (TSE) unit 218B that are configured to mechanically reduce particle size of solid particles present in the second water-based slurry 114 discharged from the first processing stage 104. The grinder mill 218A is an example of a mechanical particle size reducer configured to grind and/or mill the larger sized particles to reduce their size. As shown in FIG. 2, the system 100 in an embodiment can include the grinder mill 218A and the TSE unit 218B that are configured to mechanically reduce particle size of solid particles present in second water-based slurry 114. In an embodiment, each of the grinder mill 218A and the TSE unit 218B is in fluid communication with slurry recirculation 216 via flow path 220 and is in fluid communication with slurry pump inlet(s) 214 via output flow path 222. In an embodiment, the grinder mill 218A and/or the TSE unit 218B may be configured to provide an output 224 to the second processing stage 106, or are configured to provide an output 222 to slurry pump inlet(s) 214, or are configured to provide both an output 224 to the second processing stage 106 and to provide an output 222 to slurry pump inlet(s) 214. In the illustrated embodiment, the system 100 includes both the grinder mill 218A and the TSE unit 218. In another embodiment, the system 100 may include just one of the grinder miller 218A and the TSE unit 218. In another embodiment, the TSE unit 218B may be included in the second processing stage 106 (e.g., the TSE unit 218B may be the TSE 610 in Figure 3, as described below). Operation of TSE unit is discussed in more detail below.

### Second processing stage

With continued reference to FIG. 1, the system 100 comprises a second processing stage 106 in fluid communication with the first processing stage 104. The second processing stage 106 receives the second water-based slurry 114. The second processing stage 106 is configured to reduce the particle size of at least a portion of the solid particles having the particle size larger than the particle size suitable for efficient anaerobic bacterial digestion, forming a third water-based slurry 116.

FIG. 3 shows a schematic diagram of the second processing stage 106, according to an embodiment. The second processing stage 106 comprises an electrocoagulation (EC) unit 602, a Thermal Steam Explosion (TSE) unit 610, or both an EC unit 602 and a TSE unit 610.

When present, the EC unit 602 electrochemically induces hydrolysis of the second water-based slurry 114. The slurry enters an inlet in the EC unit 602 and passes between low voltage metal electrode plates (not shown), creating ions by electrolysis of the water present in the slurry. The ions destabilize the binding forces holding the solids in suspension causing particles of every size to settle out of the slurry. The rate of settling is directly proportional to the particle size and mass (i.e., particles of greater mass will settle at a higher velocity than particles of lesser mass). The destabilized solids are then directed to a collection basin, and may optionally be further processed.

The electrolysis further produces a small amount of iron ions which bind to and cause certain organic compounds to become insoluble. These insoluble compounds float out for subsequent removal.

The EC unit 602 also sanitizes the slurry through electrochemical inactivation of live microbes and viruses. Such sanitizing reduces pathogens such as viruses and bacteria including fecal species (e.g., *Enterococcus sp., E. coli,* and the like) prior to digestion.

The electrochemically induced particle separation also produces hydrolysis of solid particles which is a rate-limiting step in anaerobic digestion. Accordingly, hydrolysis occurring in the EC unit 602 accelerates methane generation in the subsequent digestion of said particles.

In an embodiment, the EC unit 602 can operate at a lower power level than others known in the art. The lower power results in a slower overall flow rate of slurry, which allows finer control of the particle settling. The slower flow rate also advantageously reduces electrolytic hydrogen formation to at or near zero, allowing the EC unit 602 to be covered, in contrast to prior EC units in the art.

Accordingly, in an embodiment, the second processing stage comprises the EC 602 unit configured to one or more of: electrochemically hydrolyze at least a portion of the solid particles having the particle size larger than the particle size suitable for efficient anaerobic bacterial digestion, electrochemically destabilize at least a portion of the solid particles having the particle size larger than the particle size suitable for efficient anaerobic bacterial digestion, and/or cause at least a portion of the solid particles having the particle size larger than the particle size suitable for efficient anaerobic bacterial digestion to stratify and settle out of the second water-based slurry.

In an embodiment, the EC unit 602 can include a collection basin 604. The collection basin 604 can include three outlets (e.g., a high outlet, a middle outlet, and a low outlet) which are utilized for tuning the EC unit 602 for optimal electrochemical separation. Each outlet is spaced apart vertically to separate solids based on their settling velocities. The smallest particles settle slowly, and hence travel directly to the high outlet. The mid-sized particles settle more rapidly than the small particles, and will travel around an internal baffle to the middle outlet. The largest particles will settle the most rapidly, moving quickly to the sloping floor of the collection basin, then through the low outlet. The EC low outlet (e.g., outlet 606) is the primary discharge for all of the flows. The high outlet and the middle outlet are primarily used for particle size measurements to tune the EC unit 602. For example, power levels to the electrode plates are adjusted to render the smallest size particles at the low outlet.

When present, the TSE unit 610 uses steam to hydrolyze, disintegrate, and sterilize waste sludge into a readily digestible product. Generally, the waste material (is heated by direct injection of steam under pressure in a reactor to a temperature in a range from about 150-230°C, at a pressure of up to about 6 bar, for a period of time ranging from about 10 to about 60 minutes. At this temperature and pressure, organic matter is hydrolyzed into soluble compounds. The hydrolyzed waste material is then expelled from the reactor and into a flash tank using the available steam pressure. The ensuing rapid pressure drop causes steam explosion, or pressure-drop disintegration, of cells and fibers comprising the solid waste particles. The resulting product is a sterilized liquid in an easily digestible form. Accordingly, an embodiment, the second processing stage 106 includes a TSE unit 610 configured to hydrolyze at least a portion of the solid particles having the particle size larger than the particle size suitable for efficient anaerobic bacterial digestion.

The TSE unit 610 is shown in FIG. 3 as treating the waste material (i.e., the second water-based slurry 114) after it has been treated by the EC unit 602. In another embodiment, the TSE unit 610 may treat the waste material before the EC unit 602 (e.g., the treated waste material discharged from the TSE unit 610 is then further treated by the EC unit 602).

Whether the EC unit and/or TSE unit are used, the effluent from the second processing stage 106 is a third water-based slurry 116, which is further enriched with respect to particles suitable for efficient anaerobic digestion relative to the first water based slurry 102 and second water-based slurry 114.

### Third processing stage

With continued reference to FIG. 1, the system 100 comprises a third processing stage 108 in fluid communication with the second processing stage 106 to receive the third water-based slurry 116. The third processing stage 108 is configured to allow passage therethrough of the waste stream to produce a fourth water-based slurry 118, which is enriched in solid particles having a particle size suitable for efficient anaerobic bacterial digestion.

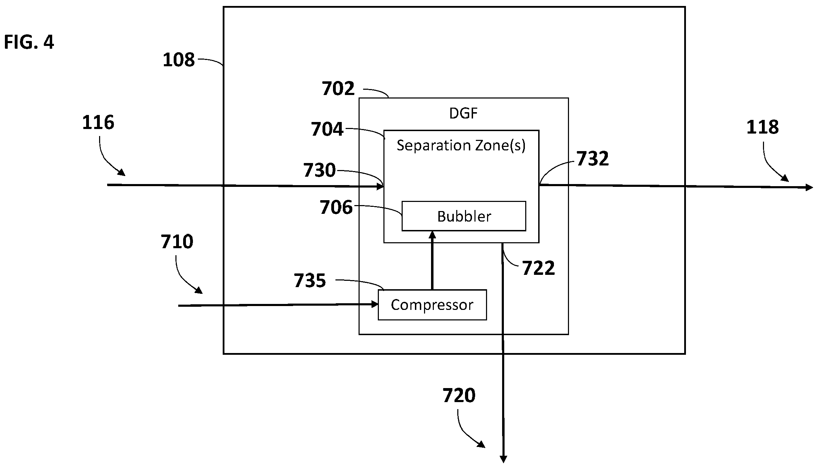

FIG. 4 shows a schematic diagram of the third processing stage 108, according to an embodiment. The third processing stage 108 comprises a dissolved gas flotation (DGF) separator 702 comprising at least one separation zone 704 comprising a bubbler 706. The DGF separator 702 is configured to remove from the third water-based slurry 116, within the at least one separation zone solid 704, particles having a particle size larger than the particle size suitable for efficient anaerobic bacterial digestion.

The DGF separator 702 and its bubbler 706 utilize a non-oxygenated gas 710 that contains substantially no oxygen (i.e., containing zero oxygen or only trace amounts of oxygen). In one example, the DGF 702 utilizes CO₂ as the non-oxygenated gas. In such an embodiment, the DGF separator 702 can be a dissolved carbon-dioxide float ("DCDF") separator and the bubbler 706 can be a CO₂ bubbler. In another embodiment, the DGF 702 may utilize a different non-oxygenated gas that includes one or more of, but is not limited to, nitrogen, methane, biogas, or the like.

For example, the DGF separator 702 can use non-oxygenated gas from a downstream scrubbing process (described below in more detail) to remove particles having a size larger than that suitable for efficient anaerobic digestion from third water-based slurry 116. The DGF separator 702 may also remove phosphorus-containing particles and non-digestible or non-methane-contributing particles. Conventional flotation devices known in the art are dissolved air flotation, which use air to float out larger particles. Air generally inhibits anaerobic digestion. The DGF separator 702 can advantageous utilize non-oxygenated gas, which does not interfere with anaerobic digestion. Further, the presence of exogenous CO₂ in the feedstock has been demonstrated to increase methane generation.

Generally, in the DGF separator 702, the non-oxygenated gas 710 enters the gas inlet, is pressurized by a compressor 735, and enters the gas bubbler 706. Fine bubbles of the non-oxygenated gas are formed by means well known in the art. The non-oxygenated gas bubbles enter the one or more separation zones 704, each zone capable of adjusting bubble flow, causing large particles present in third water-based slurry 116 to float upward, forming a scum on the surface of the fluid. Heavier particles move/fall more quickly and will drop out of suspension as sludge 720.

In an embodiment, the DGF separator 704 can include an influent manifold, one or more adjusting baffles, a sludge hopper, conveyor, and associated inlets and outlets. The third water-based slurry 116 enters an influent inlet 730 and flows through the influent manifold, travelling upward and then laterally through at least one, and preferably a plurality, of adjusting baffles (not shown). The influent then enters the one or more separation zones, where large, heavy particles settle downward through the sludge hopper and enter a sludge manifold. The flow of sludge 720 exits the DGF 702 by a sludge outlet 722. The adjusting baffles are adjustable for cross-sectional flow area, so as to regulate the flow rate of the influent (i.e., the third water-based slurry 116). In this manner, the hydrodynamic trajectory of the influent particles and the residence time in the separation zone(s) 704 may be adjusted to cause large particles to separate from the influent flow and move downward. Smaller, lighter particles (e.g., less than about 150 or 200 microns), will flow through the separation zone(s) 704 and into a clear well. Effluent flow (fourth water-based slurry 118), enriched in solid particles having a particle size suitable for efficient anaerobic bacterial digestion, exits the DGF separator 702 by an effluent outlet 732. In some embodiments, effluent pH is monitored by, e.g., a pH meter. In the event that there is an issue downstream of the DGF separator, effluent flow exiting the DGF separator 702 can be diverted to a lagoon to be stored and recycled for process water. In the event that heavy particles are found in the effluent flow exiting the DGF separator, a counter flow can be diverted to a lagoon for recycling.

The third processing stage 108 is configured to retain separated solid particles having the particle size larger than the particle size suitable for efficient anaerobic bacterial digestion, referred to herein above as sludge. In an embodiment, the scum is skimmed and collected, and moves downward into a sludge manifold to mix with the sludge 720. In an embodiment, the sludge, 720, which includes mixed heavy solids, may be returned to the first processing stage 102 for further processing. In an embodiment, the sludge 720 can collected in a tank as solids containing phosphorus.

The effluent from the third processing stage 108 is a fourth water-based slurry 118, which is enriched in the solid particles having a particle size suitable for efficient anaerobic bacterial digestion.

### Biogas production unit

With continued reference to FIG. 1, the system 100 includes a biogas production unit 110, connected in fluid communication with third processing stage 108 to receive as a feedstock the fourth water-based slurry 118. For example, the biogas production unit 110 may be in fluid communication with the DGF separator 702 of the third processing stage 108. The biogas production unit 110 is configured to anaerobically digest the solid particles in the feedstock, thereby forming at least the biogas, wastewater, and settled solids.

The biogas production unit 110 includes at least one anaerobic digester 300. The anaerobic digester 300 may be a parallel flow anaerobic digester (PFAD). One example of an anaerobic digester and a PFAD is a low solids anaerobic digester (LSAD). The anaerobic digester 300 includes one more bio-substrate(s) 304 for growing bacteria under anaerobic conditions, thereby producing the biogas.

In an embodiment, a bio-substrate 304 may be a biocurtain. In an embodiment, the anaerobic digester 300 has a horizontal flow design. The horizontal flow configuration can reduce installation cost by working at a single level, and can reduce operating cost by not requiring the pumping of viscous fluids upward for processing. Further, thermal losses can be advantageously minimized by installation of the digester below ground, in which soil serves as insulation. Further, the horizontal configuration permits the simultaneous control of solids retention time (SRT) and recirculation. The rigid, vertically mounted, high surface area biocurtain(s) provides a lateral flow of the wastewater feedstock 118, thereby allowing lighter and smaller organics to remain in the flow stream. Thus, larger solids can settle downward to the tank bottom to become settled solids.

In an embodiment, the digester 300 has a fixed film design, in which bacteria reside on permanent structures. Once the bacteria are introduced, they are fixed (i.e., permanently reside in the digester). The film of the fixed film refers to the adhesive coating (i.e., biofilm) generated by the bacteria which confines them to the digester bio-substrate(s) 304. In contrast, conventional digesters generally have the bacteria reside in the moving solids or effluent.

In the fixed film anaerobic digester, the bacteria grow on rigid walls (i.e., the bio-substrates 304) that permit them to consume organic particles floating by, to reproduce and grow in quantity and size. When the bio-substrate(s) 304 are biocurtain(s), the bacterial grow on the rigid vertical walls of the biocurtain(s). This configuration precludes large particles blocking digestion of small particles, which occurs in conventional digesters. The length of time it takes for feedstock to completely flow through the digester and exit as effluent is referred to as the hydraulic residence time (HRT). Conventional digesters have an HRT of up to 3 weeks. In contrast, the anaerobic digester disclosed herein can have a hydraulic residence time of about 3 days. The Solids Residence Time (SRT) can be varied, and is determined by monitoring the COD destruction seen in the recirculation of settled solids, as described herein below. The SRT permits retreatment of settled solids, thereby freeing up more COD. HRT is also about 3 days (e.g., less than 1 week, less than 5 days, less than 4 days). Accordingly, and advantageously, the anaerobic digester disclosed herein converts organic waste matter to methane approximately seven times faster than conventional systems.

Conventional fixed film digesters are sensitive to clogging due to excessive biofilm thickness and high suspended solids concentration in the wastewater feedstock. The presently disclosed system advantageously overcomes this problem through the solids separation occurring in the three processing stages (first processing stage 104, second processing stage 106, third processing stage 108) as described herein above, and by virtue of the disclosed anaerobic digester design (e.g., having rigid, vertically mounted, high surface area biocurtain(s), which provide a lateral flow of the wastewater feedstock, thereby allowing lighter and smaller organics to remain in the flow stream).

The anaerobic digester(s) 300 are contained within one or more digester tanks. In an embodiment, the digester tank(s) are constructed of R32 insulated concrete. The digesters can be covered with a double HDPE cover that provides an insulating air layer, so as to maintain a constant system temperature. In an embodiment, all PFAD structure and components are removable, allowing for easy installation and maintenance. Each digester tank has first and second opposed sidewalls extending from an inlet endwall to an outlet endwall. Each digester tank includes a bottom extending between the first and second sidewalls, and extending between the inlet and outlet endwalls. The bottom slopes downward from adjacent the first sidewall to adjacent the second sidewall.

The at least one anaerobic digester 300 includes at least a first bio-substrate for growing bacteria under anaerobic conditions. The bio-substrate can be a rigid substrate or wall installed inside the digester to provide the environment for anaerobic bacterial growth. Biocurtains are rigid, vertical walls installed inside the PFAD to provide the environment for anaerobic bacterial growth. Each biocurtain has two sheets of a polymeric material spaced apart at a predetermined distance. The biocurtain sheet material is generally made of a polymeric resin such as polyethylene, polypropylene, polyethylene terephthalate (PET), or the like. These materials are merely exemplary, and should be understood to be non-limiting. Conventional biocurtain sheet material is typically smooth. By contrast, the sheet material of the biocurtain in the system 100 has alternating ridges and furrows, which provides a patterned or convoluted surface that results in increased the surface area for bacterial growth. The ridges can have a predetermined, maximum ridge height so as to not occlude the release of biogas excreted by the bacteria residing on the surface of the biocurtain.

The sheets are attached to a float pipe, and depend downward from the float pipe. The float pipe floats upon the surface of the wastewater feedstock to support the biocurtain. A hanger pipe is co-extensive with the float pipe. The hanger pipe is unitary with the float pipe on an upper surface thereof. An anchor cable is received in the hanger pipe. The opposite ends of each anchor cable are secured in pockets spaced apart along upper edges of the sidewalls, to anchor the biocurtain(s). Each biocurtain sheet extends from an upper edge, attached by fasteners to the float pipe, downward to a lower edge anchored to the tank bottom by a floor bracket.

In an embodiment, the anaerobic digester(s) comprises a plurality of biocurtains spaced apart from an inlet endwall to an outlet endwall. In an embodiment, every other biocurtain can extend from a proximal end adjacent the first sidewall to a distal end adjacent the second sidewall and spaced apart from a second sidewall by a predetermined flow space. Each remaining biocurtain extends from a proximal end adjacent the second sidewall to a distal end adjacent the first sidewall and spaced apart from the first sidewall by the flow space. Each biocurtain flow space adjacent the distal end extends from the tank bottom to the wastewater surface. The biocurtains and spaces alternate so as to provide a convoluted, preferably serpentine flow of wastewater feedstock 118 through the anaerobic digester(s) 300. The flow of wastewater feedstock 118 through the anaerobic digester 300, and around the biocurtains, can assume any path or flow pattern, and the path is non-limiting.

In an embodiment, some of the wastewater influent flow of wastewater feedstock 118 can be diverted to a sweep jet inlet manifold. Sweep jet nozzles are arrayed along the length of the sweep jet inlet manifold. The sweep jet nozzles direct the feedstock118 across the tank bottom as a sweep jet flow. This stirs up and mixes with the settled solids, to become a settled solids flow, which is drawn by the settled solids nozzles into a settled solids outlet manifold. A settled solids pump pulls suction on the settled solids outlet manifold. The settled solids particles are reduced in size by fluid shear in the settled solids pump. In an embodiment, settled solids may be recycled through one or more of the three processing stages 104, 106, 108, resulting in an increase in biogas production and system process efficiency. Recirculation and comminution of settled solids provides the ability to control the Organic Loading Rate, provides pH buffering, increases hydrolysis, and increases digester stability. The anaerobic digester SRT can be varied independently, or decoupled, with respect to the HRT by means of the settled solids recirculation. Generally, the settled solids are discharged from the biogas production unit 110 in the form of a fifth water-based slurry 120. The fifth water-based slurry 120 may be monitored for particle size reduction, and corresponding increases in COD. In order to maintain SRT, ORL, pH, particle size, and COD balance, a certain percentage of the settled solids effluent may be discharged into a lagoon as a buffer.

In an embodiment, the at least one anaerobic digester may include a second bio-substrate, wherein the first and second bio-substrates are configured to independently receive, respectively, a first and second feedstock flow stream. In such an embodiment, the first feedstock flow stream and the second feedstock flow stream can each comprise a different flow rate, a different distribution of particle sizes, or both.

In an embodiment, at least two anaerobic digesters are included, i.e., a first anaerobic digester and a second anaerobic digester. The dual anaerobic digester design allows operation under a variety of flow configurations. In some embodiments, a parallel flow setup is provided, wherein feedstock flows through each anaerobic digester at about the same rate. The parallel flow setup allows maximization of the feedstock flow rate through the gas production unit 110. In an embodiment, bypasses can be provided for independently changing flow rates to the two anaerobic digesters.

Within the anaerobic digester(s) 300, methane is continuously generated by the interaction of digestible solid particles and dissolved organic compounds with specialized anaerobic bacteria. The anaerobic digester(s) are operated in the absence of oxygen operating at a temperature in a range from at or about 68°F to at or about 140°F. In an embodiment, the anaerobic digester(s) can operate in the Mesophilic range (from at or about 68°F to at or about 112°F). The heat required to maintain the preferred temperature can be provided by one or more heat exchangers 302A, 302B, 302C. In an embodiment, the heat exchanger(s) 302A, 302B, 302C are configured to provide an anaerobic digester operating temperature in a range from at or about 68 to at or about 140°F. In an embodiment, the heat exchanger(s) 302A, 302B, 302C are configured to provide an anaerobic digester operating temperature in a range from about 95 to about 130°F. For example, the heat exchangers 302A, 302B, 302C are located external to the anaerobic digester 300 and heat the feedstock 118 flowing into the anaerobic digester 300.

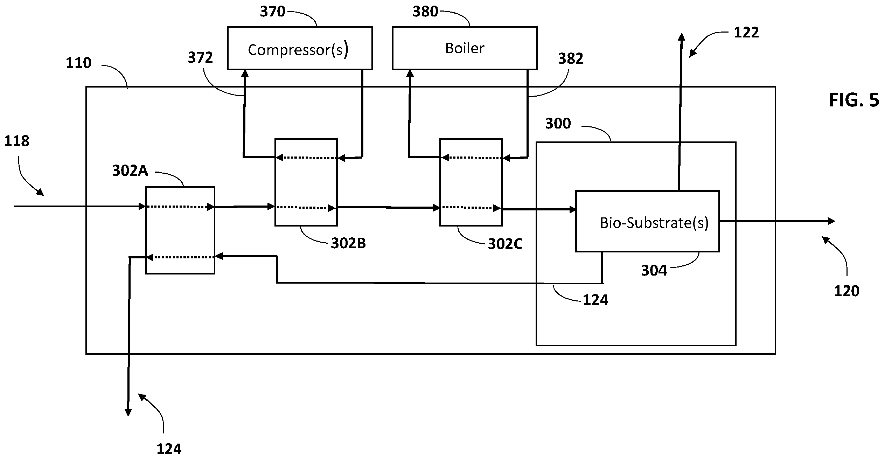

In an embodiment, the one or more heat exchangers 302A, 302B, 302C include a first heat exchanger 302A. The first heat exchanger 302A is in fluid communication with the feedstock 118 and in separate fluid communication with the effluent 124, and is configured to heat the feedstock 118 using sensible heat in the effluent 124. With reference to FIG. 5, the biogas production unit 110 includes an anaerobic digester 300 comprising at least one bio-substrate 304, each as described herein above. With continued reference to FIG. 5, the anaerobic digester 300 includes a first heat exchanger 302A, in separate fluid communication with the effluent 124 and configured to heat the feedstock 118 using sensible heat in the effluent 124. The first heat exchanger 302A transfers heat from the effluent 124 to the feedstock 118. Suitable designs for such heat exchangers are known in the art.

In an embodiment, the waste digestion system 100 includes at least one compressor 370 and a second heat exchanger 302B in fluid communication with the feedstock 118 and in separate fluid communication with the compressor(s) 370. The compressor(s) 370 can be one or those included other stages of the system 100. For example, the compressor(s) 370 may include, but is not limited to, the compressor 735 in the DGF 702, the compressor 190 in the gas refining unit 111, or the like. The second heat exchanger 302B is in fluid communication with the compressor(s) 370 via a compressor heat exchange fluid 372 that is configured to absorb waste heat from the compressor(s) 370. The second heat exchanger 302B is configured to heat the feedstock 118 using heat present in the compressor heat exchange fluid 372. Any suitable heat exchange fluid may be used, such as a glycol fluid or the like. The compressor heat exchange fluid 372 absorbs waste heat from the compressor. The feedstock 118 and the heated compressor heat exchange fluid each flowing through the second heat exchanger 302B without physically mixing with each other. As they each flow through the second heat exchanger 302B, the feedstock 118 adsorbs heat from the compressor heat exchange fluid 372, which cools the compressor heat exchange fluid. The flows through the second heat exchanger 302B can assume a variety of flow field patterns commonly used in heat exchangers, including but not limited to a flat spiral pattern. The compressor heat exchange fluid 372 after passing through the second heat exchanger 302B and being cooled by the feedstock 118 within the second heat exchanger 302B is returned to the compressor(s) 370.

In an embodiment, the waste digestion system 100 includes a boiler 380 and a third heat exchanger 302C in fluid communication with the feedstock 118 and in separate fluid communication with the boiler 380. A boiler heat exchange fluid 382 absorbs heat from the boiler 380. The third heat exchanger 302C is configured to heat the feedstock 118 using heat present in the boiler heat exchange fluid 382. Any suitable heat exchange fluid may be used, such as a glycol fluid or the like. The heat exchange fluid absorbs heat from the boiler 380 (e.g., is heated within the boiler 380). The feedstock 118 and the heated boiler heat exchange fluid 382 each flow through the third heat exchanger 302A without physically mixing with each other. The fluid tubing can assume a variety of flow field patterns commonly used in heat exchangers, including but not limited to a flat spiral pattern. The boiler heat exchange fluid 382 after passing through the third heat exchanger 302C and being cooled by the feedstock 118 within the third heat exchanger 302C is returned to the boiler 380.

The first, second, and third heat exchangers 302A, 302B, 302C may each be operated independently of one another. The first, second, and third heat exchangers 302A, 302B, 302C may be employed with one another in any combination (e.g., first and second, first and third, first, second and third, etc.). Generally, the heat exchangers are employed as necessary to maintain the desired operating temperature of the anaerobic digester 300, and the amount of heat for maintaining the operating temperature may vary based on, for example, climate conditions. Further, the location and configuration of the second and third heat exchangers 302B, 302C within the biogas production unit 110 may vary relative to the first heat exchanger 302A and relative to one another. For example, in one non-limiting embodiment, the second heat exchanger 302B is located adjacent to the biocurtain 304, and is configured to provide heat more directly to the biocurtain 304. In another embodiment, the second heat exchanger 302B can be located either upstream or downstream from the first heat exchanger 302A, and is configured to heat the feedstock 118 prior to the feedstock reaching the biocurtain 304.

The third heat exchanger 302C may be located in various positions relative to the first and second heat exchangers 302A, 302B. In an embodiment, the third heat exchanger 302C is located either upstream or downstream from the first heat exchanger 302A, and is configured to heat the feedstock prior to the feedstock reaching the biocurtain 304. In another embodiment, the third heat exchanger 302C is located downstream of the first heat exchanger 302A and upstream of the biocurtain 304.

With continued reference to FIG. 1, during operation of the system 100, effluent 124 exits the biogas production unit 110 as wastewater substantially free of solid particulate matter. After exiting the biogas production unit 110, the effluent 124 may be conveyed to a collection pit, or may flow by gravity or be pumped to a lagoon. In some embodiments, the effluent 124 is allowed to clear, and then is sent to an EQ pit or sand lane (e.g., the EQ pit 202, the sand lane for the first processing stage 104, or the like).

With continued reference to FIG. 1, during operation of the system 100, the settled solids exit the unit 110 as a fifth water-based slurry 120, and the biogas 122 exits the unit 110 as a product. The biogas 122 includes methane. The biogas 122 can also further include carbon dioxide, water vapor, heavy hydrocarbons, and/or other contaminant liquids and vapors. It may be desired to provide higher purity methane. In an embodiment, the system 100 can include a gas refining unit 111 configured to purify the biogas 122 (i.e., remove non-methane components from the biogas 122). The gas refining unit 111 can include a membrane module (not shown) in fluid communication with the anaerobic digester 300. The membrane module is configured to remove heavy hydrocarbons, CO₂, and contaminant liquids from the biogas, and configured to allow passage therethrough of methane. In an embodiment, the CO₂ bubbler 706 in the DGF separator 702 of the third processing stage 108 is in fluid communication with the membrane module via a manifold, and the manifold is configured to receive CO₂ from the membrane module.

In some embodiments, a portion of the CO₂ from the membrane module is added into a recirculation line (not shown) and directed back into the anaerobic digester 300 to enhance methane generation. In some embodiments, a portion of the CO₂ from the membrane module is dissolved in the biogas production unit effluent flow 124. Such dissolved CO₂ can be used to produce food-grade CO₂ for sale, or to grow algae and duckweed for co-digestion or as a feed supplement.

In an embodiment, the gas refining unit 111 can include a Knock Out Pot (KOP) (not shown) downstream of the anaerobic digester(s) 300. Raw biogas 122 exits the biogas production unit 110 and enters the KOP at a KOP inlet. The KOP removes very fine droplets of water entrained in the biogas and removes any liquid carryover in the biogas. Liquid exits the by a KOP effluent outlet, and is sent to a collection pit. Dry biogas leaves via a KOP gas outlet. In an embodiment, the gas refining unit 111 can include a scrubber or scrubbing system (not shown), configured to remove water vapor, particulate matter, and contaminant gas from the biogas. From the KOP outlet, raw biogas is sent to the scrubber or scrubbing system (e.g., using a blower or compressor). In an embodiment, the scrubber can be a commercially available product, such as the Series 77V Coalescing Filter, manufactured by PECOFacet. The scrubber may include an activated charcoal trap. In an embodiment, another scrubber may be included, such as a scrubber configured to remove hydrogen sulfide. A non-limiting example of one such suitable scrubber is an Iron Sponge. Generally, clean gas enriched in methane exits a scrubber outlet. In an embodiment, the resulting purified biogas is 95% - 98.5% methane. Any coalesced liquid drains through a scrubber effluent outlet and is directed to a collection pit. Excess biogas, or excess pressure which may develop in the biogas production unit 110 can be rerouted and burned off by a flare skid.

In some embodiments, the gas refining unit 111 may include a compressor 190 configured to compress the purified biogas. In some embodiments, the gas refining unit 111 can include instrumentation in fluid communication with the membrane module and configured to monitor the biogas composition, wherein said instrumentation is optionally in communication with the process control, described further herein below. In some embodiments, the biogas quality is monitored by instrumentation including, but not limited to, a gas chromatograph, to ensure consistency of the final gas composition.

As described herein above, the biogas enters the membrane module prior to a Knock Out Pot and/or scrubber. In another embodiment, the biogas may first undergo treatment with the Knock Out Pot and/or the scrubber prior to the membrane module. Accordingly, it should be understood that the biogas flow path is not limited to with respect to the order of processing.

The system 100 comprises a process control 112 that is operatively connected to the waste digestion system 100 and configured to control the waste digestion system 100. Particularly, the process control 112 is configured to monitor and control various system operating parameters, such as flow rates and flow paths, temperatures, pump operations, and the like. With continued reference to FIG. 1, process control 112 receives input signals from one or more of the first processing stage 104, the second processing stage 106, the third processing stage 108, and the biogas production unit 110. The process control 112 generally comprises a processor (not shown), a memory (memory), and input and output connections. Input signals are received from sensors or instruments throughout the system. Input signals may include pH, temperature, pressure, COD, and/or mass flow at various critical points of the system. Input signals may further include closed contacts, electrical voltage, and/or current. Output signals may be sent to devices such as flow control valves, pressure control valves, temperature control valves, diverters, and/or emergency shutdown systems (not shown) within the system 100. In an embodiment, the system 100 can include a flare skid and automatic shutoff valves to enhance safety during operation. Generally, control logic is programmed to monitor the entire process and provides the operator with ease of use and real time process status updates.

In an embodiment, the system 100 can include a pipeline injector configured to inject the produced biogas/methane 122 into a Local Distribution Company (LDC) pipeline. In an embodiment, the system 100 may be configured to supplying the produced biogas/methane to a transport vehicle (e.g., train, shipping boat, truck, or the like).

In an embodiment, the system 100 may comprise additional pumps, tanks, flow paths, lagoons, and other features, such as those illustrated in the specification and drawings of U.S. Patent No. 10,899,640, incorporated herein by reference above. Accordingly, such additional features, as well as potential layouts and configurations for the system and individual components thereof (e.g., processing stages, biogas production unit, and digester layouts) as disclosed in U.S. Patent No. 10,899,640 are contemplated herein.

### Method for generating biogas including methane

Further provided is a method for generating a biogas comprising methane from a waste stream comprising a waste material by bacterial digestion of the waste material under anaerobic conditions. The method generally comprises processing the waste material in the three processing stages as described herein, and digesting the waste material under anaerobic conditions in the biogas production unit as described herein, thereby generating biogas. Optionally the method further comprises purifying the methane-containing biogas.

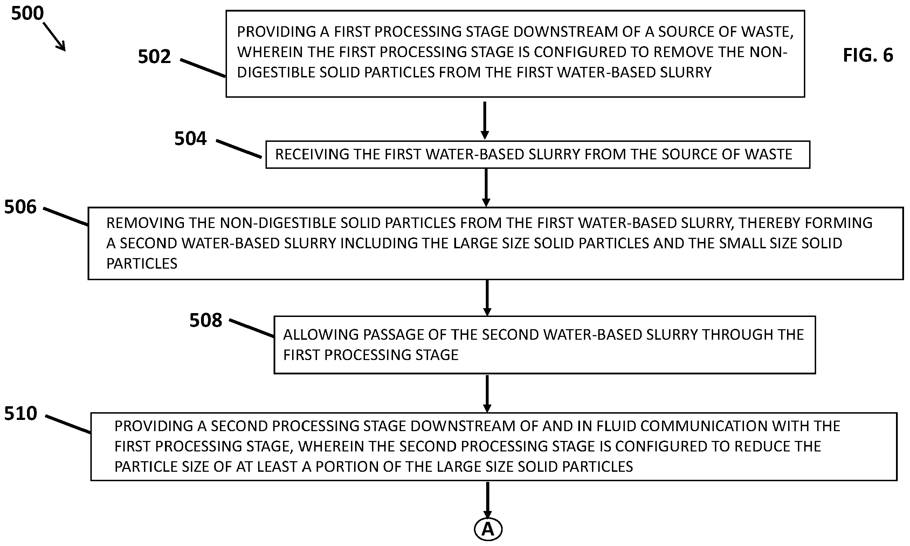

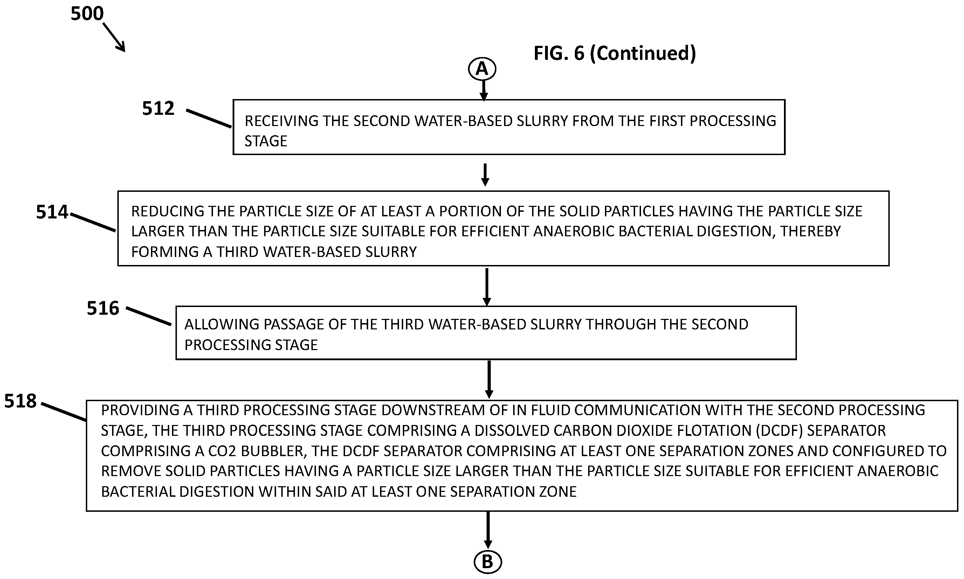

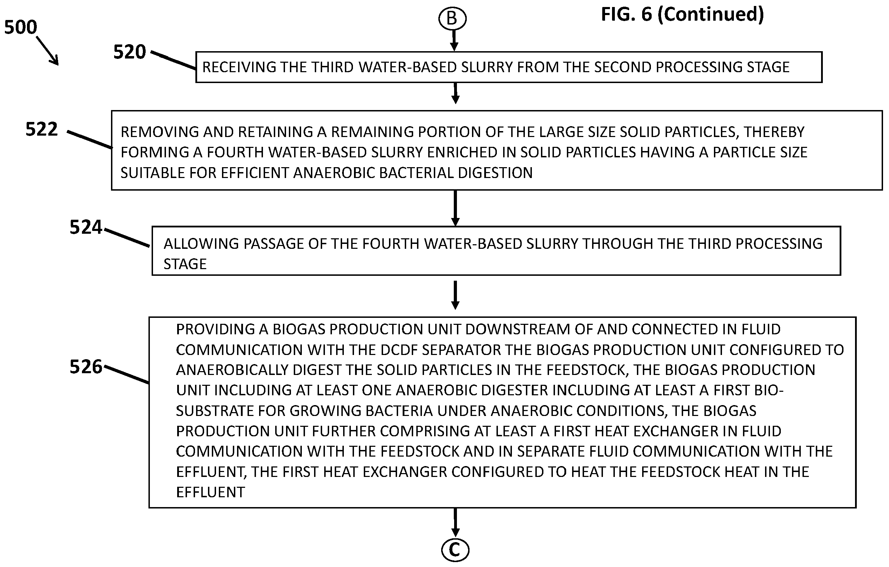

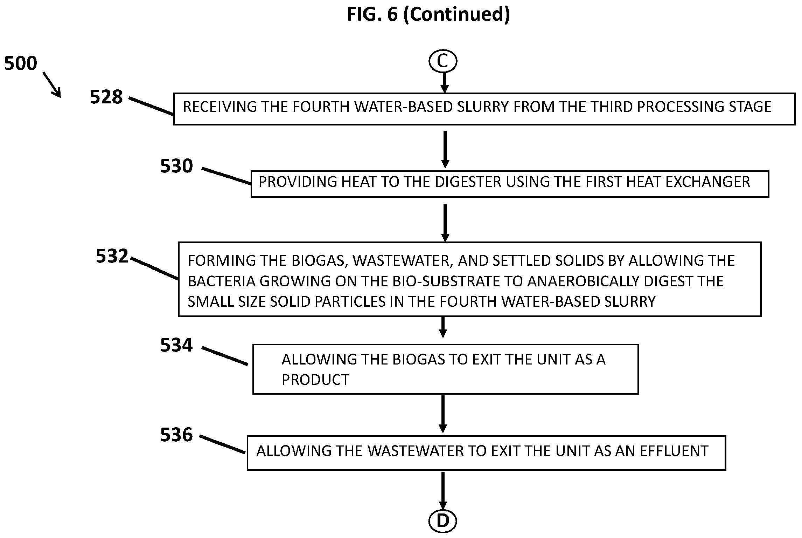

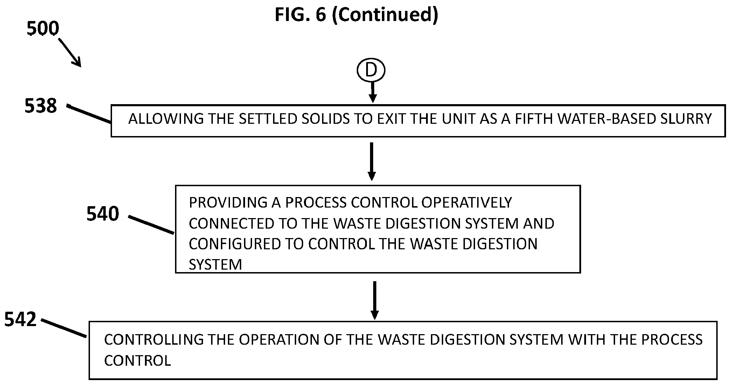

FIG. 6 is a block flow diagram of an embodiment of a method 500 of generating a biogas comprising methane from a waste stream comprising a waste material by bacterial digestion of the waste material under anaerobic conditions. The method 500 can have features as described above for the system 100.

The method 500 comprises providing a first processing stage downstream of a source of waste, wherein the first processing stage is configured to remove the non-digestible solid particles from the first water-based slurry, as shown in block 502 of FIG. 6.

The method 500 comprises receiving the first water-based slurry from the source of waste, each as described herein above, as shown in block 504 of FIG. 6.

The method 500 comprises removing the non-digestible solid particles from the first water-based slurry, thereby forming a second water-based slurry comprising solid particles having the particle size larger than the particle size suitable for efficient anaerobic bacterial digestion and solid particles having the particle size suitable for efficient anaerobic bacterial digestion, as shown in block 506 of FIG. 6.

The method 500 comprises allowing passage of the second water-based slurry through the first processing stage, as shown in block 508 of FIG. 6.

The method 500 comprises providing a second processing stage downstream of and in fluid communication with the first processing stage. The second processing stage is configured to reduce the particle size of at least a portion of the solid particles having the particle size larger than the particle size suitable for efficient anaerobic bacterial digestion, as shown in block 510 of FIG. 6.

The method 500 comprises receiving the second water-based slurry from the first processing stage into the second processing stage as described herein above, as shown in block 512 of FIG. 6.

The method 500 comprises reducing the particle size of at least a portion of the solid particles having the particle size larger than the particle size suitable for efficient anaerobic bacterial digestion, thereby forming a third water-based slurry, as shown in block 514 of FIG. 6.

The method 500 comprises allowing passage of the third water-based slurry through the second processing stage, as shown in block 516 of FIG. 6.

The method 500 comprises providing a third processing stage downstream of in fluid communication with the second processing stage, the third processing stage comprising a dissolved gas flotation (DGF) separator comprising a bubbler, the DGF separator comprising at least one separation zones and configured to remove solid particles having a particle size larger than the particle size suitable for efficient anaerobic bacterial digestion within said at least one separation zone, as shown in block 518 of FIG. 6.

The method 500 comprises receiving the third water-based slurry from the second processing stage into the third processing stage as described herein above, as shown in block 520 of FIG. 6.

The method 500 comprises removing and retaining solid particles having a particle size larger than the particle size suitable for efficient anaerobic bacterial digestion, thereby forming a fourth water-based slurry enriched in solid particles having a particle size suitable for efficient anaerobic bacterial digestion, as shown in block 522 of FIG. 6.

The method 500 comprises allowing passage of the fourth water-based slurry through the third processing stage as shown in block 524 of FIG. 6.

The method 500 comprises providing a biogas production unit downstream of and connected in fluid communication with the DGF separator the biogas production unit configured to anaerobically digest the solid particles in the feedstock, the biogas production unit comprising at least one anaerobic digester comprising at least a first biocurtain for growing bacteria under anaerobic conditions, the biogas production unit further comprising at least a first heat exchanger in fluid communication with the feedstock and in separate fluid communication with the effluent, the first heat exchanger configured to heat the feedstock using sensible heat in the effluent, as shown in block 526 of FIG. 6.

The method 500 comprises receiving the fourth water-based slurry from the third processing stage into the biogas production unit as described herein above and as shown in block 528 of FIG. 6.

The method 500 comprises providing heat to the anaerobic digester using the first heat exchanger as shown in block 530 of FIG. 6.

The method 500 comprises forming the biogas by allowing the bacteria growing on the biocurtain to anaerobically digest solid particles in the fourth water-based slurry having a particle size suitable for efficient anaerobic bacterial digestion, and further forming the wastewater and settled solids as shown in block 532 of FIG. 6.

The method 500 comprises allowing the biogas to exit the unit as a product as shown in block 534 of FIG. 6.

The method 500 comprises allowing the wastewater to exit the unit as an effluent as shown in block 536 of FIG. 6.

The method 500 comprises allowing the settled solids to exit the unit as a fifth water-based slurry as shown in block 538 of FIG. 6.

Finally, the method comprises providing a process control operatively connected to the waste digestion system and configured to control the waste digestion system as shown in block 540 of FIG. 4; and controlling the operation of the waste digestion system with the process control as described herein above and as shown in block 542 of FIG. 6.

In some embodiments, removing the non-digestible solid particles from the first water-based slurry comprises passing the first water-based slurry through one or more of the following: a passive screen, a vibratory screen, a settling chamber, a centrifugal separator, a clarifier, and a screw press. In some embodiments, the first water-based slurry is passed through at least one passive screen.

In some embodiments, the method further comprises passing the second water-based slurry through a macerator configured to reduce the particle size of at least some portion of the solid particles present in the second water-based slurry. In some embodiments, the method comprises removing from the first water-based slurry solid particles having a particle size greater than about 750 µm by passing through said passive screen.

In some embodiments, the first processing stage comprises a clarifier, the method further comprising passing the first water-based slurry or the second water-based slurry through said clarifier. Clarification is particularly useful in embodiments wherein the waste material is municipal sewage.

In some embodiments, the method comprises removing from the first water-based slurry solid particles having a particle size greater than about 750 µm; and mechanically reducing the particle size of said solid particles. For example, the solid particles may be passed through a grinder or macerator, or through a pump configured to reduce particle size through hydraulic shear.

In some embodiments, the second processing stage comprises a Thermal Steam Explosion (TSE) unit configured to hydrolyze at least a portion of the solid particles having the particle size larger than the particle size suitable for efficient anaerobic bacterial digestion, the method comprising passing the second water-based slurry through said TSE unit. In some embodiments, the method comprises flowing the second water-based slurry into a reactor in the TSE unit; injecting steam under pressure into the reactor to a temperature in a range from about 150-230°C, at a pressure of up to about 6 bar, for a period of time ranging from about 10 to about 60 minutes, thereby hydrolyzing organic matter present in the slurry into soluble compounds. The method further comprises expelling the hydrolyzed material from the reactor into a flash tank using the available steam pressure, whereby the material is subject to steam explosion, further breaking down the solid particles.

In some embodiments, the second processing stage comprises an electrocoagulation (EC) unit, the method comprising passing the second water-based slurry through said EC unit. In such embodiments, the method comprises performing one or more of the following in said EC unit: electrochemically hydrolyzing at least a portion of the solid particles having the particle size larger than the particle size suitable for efficient anaerobic bacterial digestion; electrochemically destabilizing at least a portion of the solid particles having the particle size larger than the particle size suitable for efficient anaerobic bacterial digestion; causing at least a portion of the solid particles having the particle size larger than the particle size suitable for efficient anaerobic bacterial digestion to stratify and settle out of the second water-based slurry.

In some embodiments, the system comprises one or more mechanical particle size reducers configured to mill or grind at least a portion of the solid particles having a particle size larger than a particle size suitable for efficient anaerobic bacterial digestion, and the method further comprises milling or grinding at least a portion of said solid particles by passing said particles through the one or more mechanical particle size reducers. Such milling or grinding can occur at any processing stage (e.g., in the first, second, or third processing stage), or may be performed prior to or after the waste material has passed through the three processing stages.

In some embodiments, the at least one anaerobic digester comprises a second biocurtain, the first and second biocurtains configured to independently receive, respectively, a first and second feedstock flow stream, wherein the first feedstock flow stream and the second feedstock flow stream each comprise a different flow rate, a different distribution of particle sizes, or both. In such embodiments, the method further comprises allowing the first feedstock flow stream to flow to the first biocurtain, and allowing the second feedstock flow stream to flow to the second biocurtain. The utilization of such independent flow streams allows regulation of the overall rate of biogas production. For example, it may be desirable in some embodiments to allow more feedstock to flow to a given biocurtain, and the feedstock may contain a relatively larger or smaller percentage of particles having a size suitable for efficient digestion. Depending on the volume of the waste stream, it may be desirable to direct a portion of the stream past one or more of the three processing stages in order to, e.g., trade digestion efficiency for overall processing rate. As noted herein above, it is generally preferable to provide to the biogas production unit a feedstock containing predominantly solid particles of a size suitable for efficient digestion (e.g., less than about 200, less than about 150, or even less than about 75 microns). However, larger solid particles may still be digested, albeit more gradually and hence less efficiently. Accordingly, providing a water-based slurry containing a larger relative quantity of such larger particles is further contemplated herein.

The disclosed method generally comprises providing heat to the anaerobic digester in order to maintain an elevated temperature in a range from about 68 to about 140°F. In some embodiments, the anaerobic digester temperature may be maintained in this range using heat provided by the first heat exchanger as described herein above. Particularly, the sensible heat present in the biogas production unit effluent may be extracted from and directed to the incoming feedstock by flowing the warm effluent through a suitable heat exchanger while simultaneously flowing the cooler feedstock through the heat exchanger, such that the two liquids are in a thermal transfer relationship, and allowing heat to be transferred from the warm effluent to the cool feedstock. In some embodiments, depending on climate conditions, or during initial operation, such thermal transfer may be insufficient to provide the desired operating temperature in the anaerobic digester. Accordingly, in some embodiments, additional heat sources are provided in the system, and the method comprises providing additional heat from such heat sources.

In some embodiments, the waste digestion system further comprises at least one compressor; a compressor heat exchange fluid for absorbing waste heat from the compressor; and a second heat exchanger in fluid communication with the feedstock and in separate fluid communication with the compressor, the second heat exchanger configured to heat the feedstock using heat present in the compressor heat exchange fluid. In such embodiments, the method further comprises delivering excess heat from the compressor to the feedstock by extracting heat from the compressor heat exchange fluid in the second heat exchanger. In some embodiments, the second heat exchanger is located upstream or downstream from the first heat exchanger. In some embodiments, the second heat exchanger is located adjacent to the biocurtain.

In some embodiments, in addition to or as an alternative to the compressor heat exchanger, the system comprises a boiler; a boiler heat exchange fluid for absorbing heat from the boiler; and a third heat exchanger in fluid communication with the feedstock and in separate fluid communication with the boiler, the third heat exchanger configured to heat the feedstock using heat present in the boiler heat exchange fluid. In such embodiments, the method further comprises delivering heat from the boiler heat exchange fluid, either upstream or downstream from the first and/or second heat exchanger, by extracting heat from the boiler heat exchange fluid in the third heat exchanger.

In the foregoing methods, in some embodiments, providing heat to the anaerobic digester comprises maintaining an anaerobic digester operating temperature up to about 140°F, such as in a range from about 68 to about 140°F, or from about 95 to about 130°F, or from about 95 to about 112°F.

In some embodiments, the waste digestion system further comprises a gas refining unit comprising a membrane module in fluid communication with the anaerobic digester, configured to remove heavy hydrocarbons, CO₂, and contaminant liquids from the biogas, and configured to allow passage therethrough of methane. In such embodiments, the method comprises removing the heavy hydrocarbons, the CO₂, and the contaminant liquids with the membrane module; and allowing passage of the methane through the membrane module.

In some embodiments, the waste digestion system further comprises a manifold configured to receive CO₂ from the membrane module, and the CO₂ bubbler is in fluid communication with the membrane module via the manifold. In such embodiments, the method further comprises conveying the CO₂ from the membrane module to the CO₂ bubbler in the DGF separator through the manifold. In some embodiments, the method further comprises: receiving CO₂ from the membrane module into the CO₂ bubbler; admitting CO₂ bubbles into separation zones present in the DGF separator; causing particles having a particle size larger than the particle size suitable for efficient anaerobic bacterial digestion to float upward with the CO₂ bubbles; disposing a scum hopper adjacent the separation zones, and connecting the scum hopper in fluid communication with the sludge manifold; moving the floating slurry particles having a particle size larger than the particle size suitable for efficient anaerobic bacterial digestion into the scum hopper with the conveyor blades; and conveying the particles having a particle size larger than the particle size suitable for efficient anaerobic bacterial digestion back to the first processing stage.

In an embodiment, the waste material is dairy cattle waste, and the waste digestion system further includes a sand lane for receiving the dairy cattle waste, as described herein above. In such an embodiment, the waste digestion system can further include a solids tank in fluid communication with the first processing stage, an equalization pit, and an equalization tank. The solids tank is configured to receive solid particles having the particle size larger than the particle size suitable for efficient anaerobic bacterial digestion. The equalization pit is connected in fluid communication with the sand lane and is configured to receive the sand-manure slurry and to allow passage of the first water-based slurry therethrough to the equalization tank. In such an embodiment, the method can also comprise forming a sand-manure slurry that includes receiving water from a water source and allowing the water to mix with the sand and manure, separating the bedding sand from the sand-manure slurry to form the first water-based slurry, receiving the first water-based slurry into the equalization tank, circulating the first water-based slurry in the equalization tank through a slurry pump and reducing the solid particles size by hydraulic shear within the slurry pump, and receiving solid particles having the particle size larger than the particle size suitable for efficient anaerobic bacterial digestion into the solids tank.

The above disclosure refers to small size solid particles as solid particles having a particle size suitable for efficient anaerobic bacterial digestion, with further disclosure regarding the range of particle sizes. Small size solid particles as defined herein may be interchangeably defined as solid particles having a particle size within a first particle size range. The first particle size range may correspond or be defined by any definition used herein with reference to the particle size suitable for efficient anaerobic bacterial digestion. For example, the first particle size range may be suitable for efficient anaerobic digestion (e.g., solid particles may be of a particle size suitable for efficient anaerobic digestion when the particle size is within the first particle size range ). The above disclosure refers to large size solid particles that are solid particles having a particle size larger than the particle size suitable for efficient anaerobic bacterial digestion. Large size solid particles as defined herein may be interchangeably defined as solid particles having a particle size larger than the first particle size range.

Properties discussed herein, such as particle size or the like, may be determined using known methods in the art of wastewater treatment. For example, properties of wastewater, a water-based slurry, or the like as discussed herein may be determined according to Standard Methods for the Examination of Water and Wastewater (e.g., Standard Methods for the Examination of Water and Wastewater, 24th edition, published by the American Public Health Association, American Water Works Association, and Water Environment Federation). For example, a concentration of type of solid (e.g., suspended solids, volatile solids, settleable solids, or the like) in waste stream, water-based slurry, or the like may be determined according to standard method 2540 for the Examination of Water and Wastewater. For example, a distribution of particle sizes (and/or a concentration of a particular particle size/range) in a waste stream, water-based slurry, or the like may be determined according to standard method 2560 of the Standard Methods for the Examination of Water and Wastewater.

### Aspects:

Any of Aspects 1-14 may be combined with any of Aspects 15 - 20.
Aspect 1. A waste digestion system configured to generate a biogas from a waste stream including a waste material by bacterial digestion of the waste material under anaerobic conditions, the biogas including methane, wherein the waste stream includes a first water-based slurry including solid particles, wherein the solid particles include non-digestible solid particles, small size solid particles that are solid particles having a particle size suitable for efficient anaerobic bacterial digestion, and large size solid particles that are solid particles having a particle size larger than the particle size suitable for efficient anaerobic bacterial digestion, the waste digestion system comprising: a first processing stage configured to remove the non-digestible solid particles from the first water-based slurry and to form a second water-based slurry including the small size solid particles and large size solid particles; a second processing stage in fluid communication with the first processing stage to receive the second water-based slurry, the second processing stage configured to reduce the particle size of at least a portion of the large size solid particles and to form a third water-based slurry; a third processing stage in fluid communication with the second processing stage to receive the third water-based slurry, the third processing stage including a dissolved gas flotation (DGF) separator having at least one separation zone including a bubbler, the DGF separator configured to utilize non-oxygenated gas to remove a remaining portion of the large size solid particles from the third water-based slurry, such that the third processing stage discharges a fourth water-based slurry enriched in the small size solid particles; and a biogas production unit connected in fluid communication with the DGF separator to receive the fourth water-based slurry as a feedstock, and the biogas production unit configured to anaerobically digest the small sized solid particles in the feedstock forming the biogas, wastewater, and settled solids, wherein the biogas production unit is configured to discharge the biogas as a product, to discharge the wastewater as an effluent, and to discharge the settled solids as a fifth water-based slurry, the biogas production unit including at least one anaerobic digester with a first bio-substrate for growing bacteria under anaerobic conditions to produce the biogas and a first heat exchanger configured to heat the feedstock using heat in the effluent.
Aspect 2. The waste digestion system of Aspect 1, further comprising one or more mechanical particle size reducers configured to at least one mill and grind at least a portion of the larger solid particles.
Aspect 3. The waste digestion system of any one of Aspects 1 and 2, wherein the first processing stage comprises at least one of a macerator and a grinder configured to reduce the particle size of at least a portion of the solid particles.
Aspect 4. The waste digestion system of any one of Aspects 1-3, wherein the second processing stage comprises a Thermal Steam Explosion (TSE) unit configured to hydrolyze at least a portion of the large size solid particles.
Aspect 5. The waste digestion system of any one of Aspects 1-4, wherein the second processing stage includes an electrocoagulation (EC) unit configured to perform one or more of the following: electrochemically hydrolyze at least a portion of the large size solid particles; electrochemically destabilize at least a portion of the large size solid particles; and cause at least a portion of the large size solid particles to stratify and settle out of the second water-based slurry.
Aspect 6. The waste digestion system of any one of Aspects 1-5, wherein the at least one anaerobic digester includes a second bio-substrate, wherein the first bio-substrate and the second substrate are configured to independently receive, respectively, a first feedstock stream and a second feedstock flow stream, and wherein the first feedstock flow stream and the second feedstock flow stream have at least one of a different flow rate and a different distribution of particle sizes.
Aspect 7. The waste digestion system of any one of Aspects 1 - 6, further comprising: at least one compressor; a compressor heat exchange fluid for absorbing waste heat from the compressor; and a second heat exchanger configured to heat the feedstock using the compressor heat exchange fluid heated by the waste heat of the compressor.
Aspect 8. The waste digestion system of any one of Aspect 7, further comprising: a boiler; a boiler heat exchange fluid for absorbing heat from the boiler; and a third heat exchanger configured to heat the feedstock using the boiler heat exchange fluid heated by the boiler.
Aspect 9. The waste digestion system of any one of Aspects 1 - 8, wherein the at least one heat exchanger is configured to provide an anaerobic digester operating temperature in a range from at or about 68 to at or about 140°F.
Aspect 10. The waste digestion system of any one of Aspects 1 - 9, wherein the waste material comprises one or more of: waste food, municipal sewage waste, animal waste from farming operations, industrial organic waste, and fat, oil, and grease (FOG) waste.
Aspect 11. The waste digestion system of any one of Aspects 1 - 10, further comprising: an equalization tank; at least one slurry pump for reducing particle size of the solid particles by hydraulic shear, the at least one slurry pump in fluid communication with the equalization tank, and wherein the first processing stage is in fluid communication with the equalization tank and the slurry pump; and a solids tank in fluid communication with the first processing stage, the solids tank configured to receive a portion of the large solid particles.
Aspect 12. The waste digestion system of any one of Aspects 1 - 11, wherein the particle size suitable for efficient anaerobic bacterial digestion is less than about 200 µm.
Aspect 13. The waste digestion system of any one of Aspects 1 - 12, wherein the first processing stage is further configured to remove from the first water-based slurry solid particles having a particle size greater than about 750 µm, and is further configured to mechanically reduce particle size of said solid particles.
Aspect 14. The waste digestion system of any one of Aspects 1 - 13, further comprising a process control configured to control the waste digestion system.
Aspect 15. A method for generating a biogas from a waste stream including a waste material by bacterial digestion of the waste material under anaerobic conditions, the biogas including methane, wherein the waste stream includes a first water-based slurry including solid particles, wherein the solid particles include non-digestible solid particles, small sized solid particles that are solid particles having a particle size suitable for efficient anaerobic bacterial digestion, and large size solid particles that are solid particles having a particle size larger than the particle size suitable for efficient anaerobic bacterial digestion, , the method comprising: providing a first processing stage downstream of a source of waste; receiving the first water-based slurry from the source of waste; removing, by the first processing stage, the non-digestible solid particles from the first water-based slurry, thereby forming a second water-based slurry including the small size solid particles and the large size solid particles; allowing passage of the second water-based slurry through the first processing stage; providing a second processing stage downstream of and in fluid communication with the first processing stage; receiving the second water-based slurry from the first processing stage; reducing, by the second processing stage, the particle size of at least a portion of the large size solid particles, thereby forming a third water-based slurry; allowing passage of the third water-based slurry through the second processing stage; providing a third processing stage downstream of and in fluid communication with the second processing stage, the third processing stage including a dissolved gas flotation (DGF) separator comprising a bubbler, the DGF separator including at least one separation zones; receiving the third water-based slurry from the second processing stage; removing and retaining a remaining portion of the large size solid particles in the at least one separation zone using non-oxygenated gas, thereby forming a fourth water-based slurry enriched in the small size solid particles; allowing passage of the fourth water-based slurry through the third processing stage; providing a biogas production unit downstream of and in fluid communication with the DGF separator, the biogas production unit including at least one anaerobic digester including at least a first bio-substrate for growing bacteria under anaerobic conditions and a first heat exchanger; receiving the fourth water-based slurry from the third processing stage as a feedstock to the biogas production unit; forming the biogas, wastewater, and settled solids by allowing the bacteria growing on the bio-substrate to anaerobically digest the small size solid particles in the fourth water-based slurry; allowing the biogas to exit the biogas production unit as a product; allowing the wastewater to exit the biogas production unit as an effluent, the first heat exchanger in fluid communication with the feedstock and in separate fluid communication with the effluent, the first heat exchanger configured to provide heat to feedstock using heat in the effluent; allowing the settled solids to exit the biogas production unit as a fifth water-based slurry.
Aspect 16. The method of Aspect 15, wherein removing the non-digestible solid particles from the first water-based slurry comprises passing the first water-based slurry through one or more of the following: a passive screen, a vibratory screen, a settling chamber, a centrifugal separator, a clarifier, and a screw press.
Aspect 17. The method of any one of Aspects 15 and 16, further comprising: removing from the first water-based slurry the solid particles having a particle size greater than about 750 µm; and mechanically reducing the particle size of said solid particles.
Aspect 18. The method of any one of Aspects 15 - 17, wherein the second processing stage comprises a thermal steam explosion (TSE) unit configured to hydrolyze at least a portion of the large sized solid particles, the method comprising passing the second water-based slurry through the TSE unit.
Aspect 19. The method of any one of Aspects 15 - 18, wherein the second processing stage comprises an electrocoagulation (EC) unit, the method comprising passing the second water-based slurry through said EC unit, thereby performing one or more of the following: electrochemically hydrolyzing at least a portion of the large size solid particles; electrochemically destabilizing at least a portion of the large size solid particles; causing at least a portion of the large size solid particles to stratify and settle out of the second water-based slurry.
Aspect 20. The method of any one of Aspects 15 - 19, further comprising: providing one or more mechanical particle size reducers configured to at least one of mill and grind solid particles to have a smaller size; and milling or grinding, with the one or more mechanical particle size reducers, at least a portion of the large size solid particles by passing said large size solid particles through the one or more mechanical particle size reducers.

## Claims

1. A waste digestion system configured to generate a biogas from a waste stream including a waste material by bacterial digestion of the waste material under anaerobic conditions, the biogas including methane, wherein the waste stream includes a first water-based slurry including solid particles, wherein the solid particles include non-digestible solid particles, small size solid particles that are solid particles having a particle size within a first particle size range, and large size solid particles that are solid particles having a particle size larger than the first particle size range, the waste digestion system comprising:
a first processing stage configured to remove the non-digestible solid particles from the first water-based slurry and to form a second water-based slurry including the small size solid particles and large size solid particles;
a second processing stage in fluid communication with the first processing stage to receive the second water-based slurry, the second processing stage configured to reduce the particle size of at least a portion of the large size solid particles and to form a third water-based slurry;
a third processing stage in fluid communication with the second processing stage to receive the third water-based slurry, the third processing stage including a dissolved gas flotation (DGF) separator having at least one separation zone including a bubbler, the DGF separator configured to utilize non-oxygenated gas to remove a remaining portion of the large size solid particles from the third water-based slurry, such that the third processing stage discharges a fourth water-based slurry enriched in the small size solid particles; and
a biogas production unit connected in fluid communication with the DGF separator to receive the fourth water-based slurry as a feedstock, and the biogas production unit configured to anaerobically digest the small sized solid particles in the feedstock forming the biogas, wastewater, and settled solids, wherein the biogas production unit is configured to discharge the biogas as a product, to discharge the wastewater as an effluent, and to discharge the settled solids as a fifth water-based slurry, the biogas production unit including at least one anaerobic digester with a first bio-substrate for growing bacteria under anaerobic conditions to produce the biogas and a first heat exchanger configured to heat the feedstock using heat in the effluent.

2. The waste digestion system of claim 1, further comprising one or more mechanical particle size reducers configured to at least one mill and grind at least a portion of the larger solid particles.

3. The waste digestion system of any one of claims 1-2, wherein the first processing stage comprises at least one of a macerator and a grinder configured to reduce the particle size of at least a portion of the solid particles.

4. The waste digestion system of any one of claims 1-3, wherein the second processing stage includes an electrocoagulation (EC) unit configured to perform one or more of the following:
electrochemically hydrolyze at least a portion of the large size solid particles,
electrochemically destabilize at least a portion of the large size solid particles, and
cause at least a portion of the large size solid particles to stratify and settle out of the second water-based slurry.

5. The waste digestion system of any one of claims 1-4, wherein the at least one anaerobic digester includes a second bio-substrate, wherein the first bio-substrate and the second substrate are configured to independently receive, respectively, a first feedstock stream and a second feedstock flow stream, and wherein the first feedstock flow stream and the second feedstock flow stream have at least one of a different flow rate and a different distribution of particle sizes.

6. The waste digestion system of any one of claims 1-5, further comprising:
at least one compressor;
a compressor heat exchange fluid for absorbing waste heat from the compressor;
a second heat exchanger configured to heat the feedstock using the compressor heat exchange fluid heated by the waste heat of the compressor;
a boiler;
a boiler heat exchange fluid for absorbing heat from the boiler; and
a third heat exchanger configured to heat the feedstock using the boiler heat exchange fluid heated by the boiler.

7. The waste digestion system of any one of claims 1-6, wherein the at least one heat exchanger is configured to provide an anaerobic digester operating temperature in a range from at or about 68 to at or about 140°F.

8. The waste digestion system of any one of claims 1-7, wherein the waste material comprises one or more of: waste food, municipal sewage waste, animal waste from farming operations, industrial organic waste, and fat, oil, and grease (FOG) waste.

9. The waste digestion system of any one of claims 1-8, further comprising:
an equalization tank;
at least one slurry pump for reducing particle size of the solid particles by hydraulic shear, the at least one slurry pump in fluid communication with the equalization tank, and wherein the first processing stage is in fluid communication with the equalization tank and the slurry pump; and
a solids tank in fluid communication with the first processing stage, the solids tank configured to receive a portion of the large solid particles.

10. The waste digestion system of any one of claims 1-9, wherein
the first particle size range is less than about 200 µm, and
the first processing stage is further configured to remove from the first water-based slurry solid particles having a particle size greater than about 750 µm, and is further configured to mechanically reduce particle size of said solid particles.

11. A method for generating a biogas from a waste stream including a waste material by bacterial digestion of the waste material under anaerobic conditions, the biogas including methane, wherein the waste stream includes a first water-based slurry including solid particles, wherein the solid particles include non-digestible solid particles, small sized solid particles that are solid particles having a particle size within a first particle size range, and large size solid particles that are solid particles having a particle size larger than the first particle size range, the method comprising:
providing a first processing stage downstream of a source of waste;
receiving the first water-based slurry from the source of waste;
removing, by the first processing stage, the non-digestible solid particles from the first water-based slurry, thereby forming a second water-based slurry including the small size solid particles and the large size solid particles;
allowing passage of the second water-based slurry through the first processing stage;
providing a second processing stage downstream of and in fluid communication with the first processing stage;
receiving the second water-based slurry from the first processing stage;
reducing, by the second processing stage, the particle size of at least a portion of the large size solid particles, thereby forming a third water-based slurry;
allowing passage of the third water-based slurry through the second processing stage;
providing a third processing stage downstream of and in fluid communication with the second processing stage, the third processing stage including a dissolved gas flotation (DGF) separator comprising a bubbler, the DGF separator including at least one separation zones;
receiving the third water-based slurry from the second processing stage;
removing and retaining a remaining portion of the large size solid particles in the at least one separation zone using non-oxygenated gas, thereby forming a fourth water-based slurry enriched in the small size solid particles;
allowing passage of the fourth water-based slurry through the third processing stage;
providing a biogas production unit downstream of and in fluid communication with the DGF separator, the biogas production unit including at least one anaerobic digester including at least a first bio-substrate for growing bacteria under anaerobic conditions and a first heat exchanger;
receiving the fourth water-based slurry from the third processing stage as a feedstock to the biogas production unit;
forming the biogas, wastewater, and settled solids by allowing the bacteria growing on the bio-substrate to anaerobically digest the small size solid particles in the fourth water-based slurry;
allowing the biogas to exit the biogas production unit as a product;
allowing the wastewater to exit the biogas production unit as an effluent, the first heat exchanger in fluid communication with the feedstock and in separate fluid communication with the effluent, the first heat exchanger configured to provide heat to feedstock using heat in the effluent; and
allowing the settled solids to exit the biogas production unit as a fifth water-based slurry.

12. The method of claim 11, further comprising:
providing one or more mechanical particle size reducers configured to at least one of mill and grind solid particles to have a smaller size; and
milling or grinding, with the one or more mechanical particle size reducers, at least a portion of the large size solid particles by passing said large size solid particles through the one or more mechanical particle size reducers,
wherein removing the non-digestible solid particles from the first water-based slurry comprises passing the first water-based slurry through one or more of the following: a passive screen, a vibratory screen, a settling chamber, a centrifugal separator, a clarifier, and a screw press.

13. The method of any one of claims 11 - 12, wherein the second processing stage comprises an electrocoagulation (EC) unit, the method comprising passing the second water-based slurry through said EC unit, thereby performing one or more of the following:
electrochemically hydrolyzing at least a portion of the large size solid particles,
electrochemically destabilizing at least a portion of the large size solid particles, and
causing at least a portion of the large size solid particles to stratify and settle out of the second water-based slurry.

14. The method of any one of claims 11 - 13, or the waste digestion system of any one of claims 1-10, wherein the second processing stage comprises a thermal steam explosion (TSE) unit configured to hydrolyze at least a portion of the large sized solid particles, the method comprising passing the second water-based slurry through the TSE unit.

15. The method of any one of claims 11 - 14, or the waste digestion system of any one of claims 1-10, wherein the small sized solid particles have a particle size suitable for efficient anaerobic bacterial digestion, and the particle size of the large size solid particles is larger than the particle size suitable for efficient anaerobic bacterial digestion.
